(19) Europäisches Patentamt
European Patent Office
Office européen des brevets

(11) Veröffentlichungsnummer : **0 140 243**
**B1**

(12) # EUROPÄISCHE PATENTSCHRIFT

(45) Veröffentlichungstag der Patentschrift : **09.11.88**

(21) Anmeldenummer : **84112213.8**

(22) Anmeldetag : **11.10.84**

(51) Int. Cl.[4] : **C 07 C101/42**, C 07 C101/34, C 07 C101/28, C 07 C 97/10, C 07 C103/28, C 07 C103/76, C 07 C 93/06, C 07 C 93/14, C 07 C 93/187, C 07 C143/80, C 07 D333/06

(54) **Phenoxypropanolamine.**

(30) Priorität : **19.10.83 CH 5684/83**
**07.09.84 CH 4288/84**

(43) Veröffentlichungstag der Anmeldung : **08.05.85 Patentblatt 85/19**

(45) Bekanntmachung des Hinweises auf die Patenterteilung : **09.11.88 Patentblatt 88/45**

(84) Benannte Vertragsstaaten :
**AT BE CH DE FR GB IT LI LU NL SE**

(56) Entgegenhaltungen :
**EP-A- 0 007 204**
**EP-A- 0 099 707**
**EP-A- 0 101 069**
**EP-A- 0 170 121**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**
**CH-4002 Basel (CH)**

(72) Erfinder : **Alig, Leo, Dr.**
**Liebrütistrasse 32**
**CH-4303 Kaiseraugst (CH)**
Erfinder : **Müller, Marcel, Dr.**
**Quellenweg 10**
**CH-4402 Frenkendorf (CH)**

(74) Vertreter : **Lederer, Franz, Dr. et al**
**Van der Werth, Lederer & Riederer Patentanwälte**
**Lucile-Grahn-Strasse 22**
**D-8000 München 80 (DE)**

**EP 0 140 243 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Phenoxypropanolamine, Verfahren zu ihrer Herstellung, neue Zwischenprodukte dazu und pharmazeutische Präparate auf der Basis dieser Verbindungen.

Die erfindungsgemässen Phenoxypropanolamine sind Verbindungen der Formel

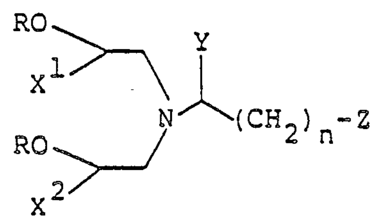

(I)

worin

n die Zahl 1 oder 2,

R Wasserstoff, nieder-Alkanoyl oder Phenyl-nieder-alkanoyl,

$X^1$ gegebenenfalls in ortho-Stellung mono-fluoriertes oder -chloriertes Phenoxymethyl,

$X^2$ nieder-Alkyl, gegebenenfalls in ortho-Stellung mono-fluoriertes oder -chloriertes Phenoxymethyl oder gegebenenfalls durch Fluor, Chlor, Trifluormethyl oder nieder-Alkoxy monosubstituiertes Phenyl,

Y Wasserstoff oder Methyl,

Z eine Gruppe der Formel

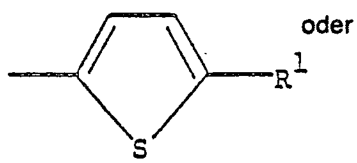

oder

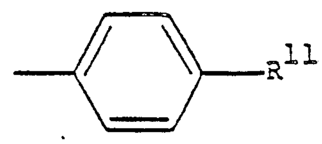

$R^1$ gegebenenfalls N-mono- oder N-di-nieder-alkyliertes Aminomethyl oder ein Rest $—C(O)R^2$,
$—C(R^3)=CH—(CH_2)_m—C(O)R^2$,
$—C(H,R^3)—(CH_2)_{m+1}—C(O)R^2$,
$—C(H,R^3)—(CH_2)_p—OH$ oder
$—C(R^3)=CH—C(CH_3)=CH—COOCH_3$,

$R^{11}$ Hydroxy, nieder-Alkanoyloxy, Sulfamoyl, gegebenenfalls durch Fluor, Chlor, Trifluormethyl, nieder-Alkyl oder nieder-Alkoxy ringsubstituiertes Benzyloxy oder Phenoxy oder eine Gruppe
$R^1$, $—O—(CH_2)_q—OH$,
$—O—(CH_2)_q—O—(CH_2)_t—R^5$ oder

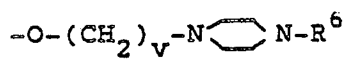

$R^2$ Hydroxy, nieder-Alkyl, nieder-Alkoxy, Dimethylaminoäthoxy, nieder-Alkoxycarbonyläthyl oder gegebenenfalls mono-oder di-niederalkyliertes Amino,

$R^3$ Wasserstoff oder Methyl,

$R^5$ Wasserstoff, nieder-Alkyl oder Phenyl,

$R^6$ nieder-Alkyl oder gegebenenfalls durch Fluor, Chlor, nieder-Alkyl oder nieder-Alkoxy para-substituiertes Phenyl,

m und p ganze Zahlen von 0 bis 6,

v eine ganze Zahl von 2 bis 4,

q und t ganze Zahlen von 1 bis 6 sind,

und physiologisch verträgliche Salze davon.

Der hier verwendete Ausdruck « nieder » bezeichnet Reste mit 1-6 Kohlenstoffatomen, wobei Reste mit 1-4 Kohlenstoffatomen bevorzugt sind. Alkyl- und Alkoxygruppen können geradkettig oder verzweigt sein. Beispiele sind Methyl, Aethyl, Propyl, Isopropyl, n-Butyl und Isobutyl bzw. Methoxy, Aethoxy, Propoxy, Isopropoxy, Butoxy und Isobutoxy. Nieder-alkanoyloxyreste leiten sich von nieder-Alkancarbonsäuren, wie Ameisensäure, Essigsäure, Propionsäure und Buttersäure ab.

Die Verbindungen der Formel I bilden mit Säuren Salze, die ebenfalls Gegenstand der Erfindung sind. Beispiele solcher Salze sind Salze mit physiologisch verträglichen Mineralsäuren, wie Salzsäure, Bromwasserstoffsäure, Schwefelsäure, Phosphorsäure ; oder mit organischen Säuren, wie Methansulfonsäure, Essigsäure, Propionsäure, Zitronensäure, Oxalsäure, Bernsteinsäure, Aepfelsäure, Fumarsäure, Phenylessigsäure oder Salicylsäure. Carbonsäuren der Formel I können als Salze vorliegen. Beispiele für solche Salze sind Alkalimetall-, Erdalkalimetall-, Ammonium- und Alkylammoniumsalze, wie Na-, K-, Ca-, Trimethylammonium- und Aethanolammoniumsalze.

Die Verbindungen der Formel I enthalten zumindest zwei asymmetrische Kohlenstoffatome und können somit als optisch aktive Enantiomere, als Diastereomere oder als Racemate vorliegen.

Die Verbindungen der Formel I können erfindungsgemäss dadurch erhalten werden, dass man

a) ein Epoxyd der Formel

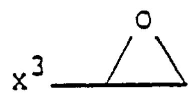
(II)

oder ein β-Ketohalogenid der Formel

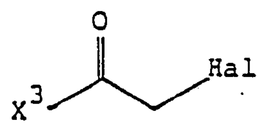
(III)

mit einem primären Amin der Formel

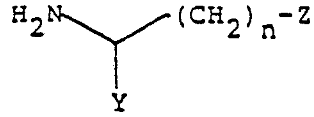
(IV)

oder einem sekundären Amin der Formel

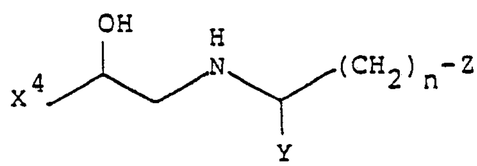
(V)

umsetzt,
wobei in den Formeln II-V n, Y und Z die obige Bedeutung haben, Hal Halogen, eine der Gruppen $X^3$ und $X^4$ eine Gruppe $X^1$ und die andere eine Gruppe $X^2$ ist, wobei ferner $X^3$ eine Gruppe $X^1$ ist, falls man eine Verbindung der Formel II oder III mit einer solchen der Formel IV umsetzt,
und eine in einer erhaltenen Verbindung vorhandene $X^1$—C(O)— oder $X^2$—C(O)-Gruppe zu einer $X^1$—CHOH— bzw. $X^2$—CHOH-Gruppe reduziert,

b) gewünschtenfalls einen in einer Gruppe Z einer Verbindung der Formel I enthaltenden reaktionsfähigen Substituenten funktionell abwandelt,

c) gewünschtenfalls die in einem Diol der Formel I vorhandenen Hydroxygruppen alkanoyliert oder phenalkanoyliert, und

d) gewünschtenfalls eine Verbindung der Formel I in ein Salz überführt.

Die Umsetzung einer Verbindung der Formel II mit einer Verbindung der Formel IV oder V kann in für die Umsetzung von Epoxiden mit Aminen zu Aminoalkoholen an sich bekannter Weise vorgenommen werden. Zweckmässig werden die Reaktionspartner in einem geeigneten Lösungsmittel zusammengebracht und erwärmt. Als Lösungsmittel kommen inerte organische Lösungsmittel, z. B. Dimethylsulfoxid (DMSO), Acetonitril oder Aether, wie Tetrahydrofuran (THF) oder Dioxan ; oder Alkohole, wie Aethanol, in Betracht. Die Reaktionstemperatur ist nicht kritisch, zweckmässig arbeitet man bei Temperaturen zwischen 60 °C und dem Siedepunkt des Reaktionsgemisches.

Die Umsetzung einer Verbindung der Formel III mit einer solchen der Formel IV oder V kann ebenfalls in an sich bekannter Weise, zweckmässig in Gegenwart eines aprotischen Lösungsmittels, wie eines halogenierten Kohlenwasserstoffs, z. B. Chloroform, bei einer Temperatur bis zu 200 °C durchgeführt werden.

Die Verbindungen der Formel

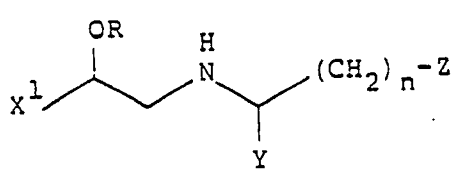
V-1

worin $X^1$, R, Y, Z und n die früher angegebene Bedeutung haben, und deren physiologisch verträglichen Salze sind neu und, sowie ein Verfahren zu ihrer Herstellung, ebenfalls Gegenstand der Erfindung. Weiterhin betrifft die Erfindung Verbindungen der Formel V-1 worin $X^1$, R, Y, Z und n die früher angegebene Bedeutung haben, sowie Verbindungen der Formel V-1, worin Z durch nieder-Alkoxy oder durch eine $—O—(CH_2)_q—COOR^4$ oder $—O—(CH_2)_q—O—(CH_2)_t—R^5$ substituiertes Phenyl ist, worin $R^4$ nieder-Alkyl und $R^5$ durch Chlor, Fluor, Trifluormethyl, nieder-Alkyl oder nieder-Alkoxy para-substituiertes Phenyl ist, als therapeutisch wirksame Mittel oder als Futterzusatz, sowie Arzneimittel auf der Basis dieser Verbindungen und die Verwendung dieser Verbindungen zur Herstellung von Arzneimitteln. Anstelle des Phenoxymethylrestes $R^1$ enthalten die in EP-A-0 007 204 beschriebenen Verbindungen einen Phenylrest. Die Verbindungen der Formel V-1 können dadurch erhalten werden, dass man

a) ein Epoxyd der Formel

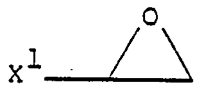

II-1

oder ein β-Ketohalogenid der Formel

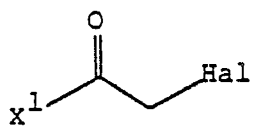

III-1

mit einem Amin der Formel IV umsetzt, und eine in einer erhaltenen Verbindung vorhandene $X^1—C(O)$-Gruppe zur $X^1—CH(OH)$-Gruppe reduziert, oder

b) eine Verbindung einer der Formeln

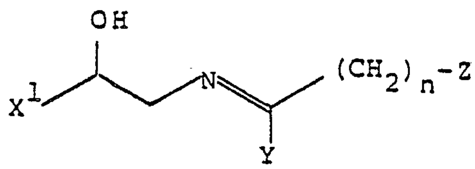

(VI)

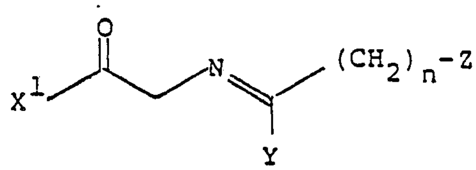

(VII)

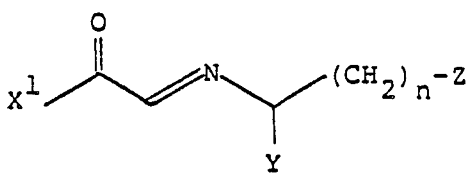

(VIII)

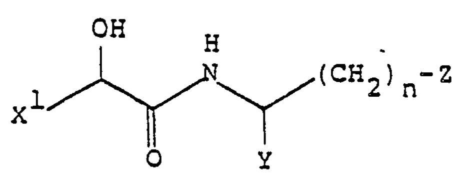

(IX)

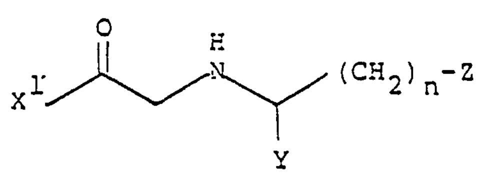

(X)

reduziert,

c) gewünschtenfalls einen in einer Gruppe Z einer Verbindung der Formel V-1 enthaltenen reaktionsfähigen Substituenten funktionell abwandelt,

d) gewünschtenfalls die in einem Alkohol der Formel V-1 vorhandene Hydroxygruppe alkanoyliert oder phenalkanoyliert, und

e) gewünschtenfalls eine Verbindung der Formel V-1 in ein Salz überführt,
wobei in den vorstehenden Formeln die Reste $X^1$, Y, Z und n die früher angegebene Bedeutung haben.

Die Umsetzung einer Verbindung der Formel II-1 oder III-1 mit einer Verbindung der Formel IV kann in einem inerten organischen Lösungsmittel, zweckmässig einem protischen Lösungsmittel, wie einem niederen Alkanol, z. B. Aethanol, vorgenommen werden. Die Reaktionstemperatur ist nicht kritisch, sie kann zwischen Raumtemperatur und der Rückflusstemperatur des Reaktionsgemisches liegen.

Die Reduktion einer Verbindung der Formel VI kann durch katalytische Hydrierung, z. B. in Gegenwart von Edelmetallkatalysatoren, wie Pd- oder Pt-Katalysatoren oder durch Behandlung mit einem komplexen Metallhydrid, wie $NaBH_4$, vorgenommen werden. Hierbei können die für derartige Reduktionen üblichen Reaktionsbedingungen angewandt werden. Die katalytische Hydrierung wird zweckmässig in einem inerten organischen Lösungsmittel, wie einem niederen Alkanol, z. B. Aethanol, bei Raumtemperatur oder schwach erhöhter Temperatur, z. B. bei 20-80 °C durchgeführt. Die Reduktion mit einem komplexen Metallhydrid wird zweckmässig in einem niederen Alkanol, z. B. Methanol, bei Temperaturen von 20-30 °C durchgeführt.

Die Verbindungen der Formeln VII, VIII, IX und X können mit einem komplexen Metallhydrid in Analogie zu den Verbindungen der Formel VI reduziert werden. Ein geeignetes komplexes Metallhydrid für die Reduktion der Verbindungen VII und VIII ist $NaBH_4$. Die Verbindungen IX werden zweckmässig mit $LiAlH_4$ reduziert.

Bei Umsetzung einer Verbindung III oder III-1 mit einer Verbindung IV oder V entstandene Ketogruppen $X^1$—C(O)— bzw. $X^2$—C(O)— können in an sich bekannter Weise zu den sekundären Alkoholgruppen reduziert werden. Diese Reduktion kann unter den gleichen Bedingungen wie die weiter oben beschriebene Reduktion der Verbindungen VI-X durchgeführt werden, wobei die Reduktion mit einem komplexen Metallhydrid, insbesondere $NaBH_4$, auf Grund ihrer Selektivität bevorzugt ist.

In dem so erhaltenen Reaktionsprodukt der Formel I oder V-1 kann ein reaktionsfähiger Substituent, insbesondere eine Gruppe —C(O)$R^2$ oder —C($R^3$)=CH—$(CH_2)_m$—C(O)$R^2$, funktionell abgewandelt werden. Die Veresterung einer Carboxylgruppe kann in an sich bekannter Weise, z. B. mittels Alkylhalogeniden, wie Methyljodid, und einer Base vorgenommen werden. Die Verseifung einer Estergruppe wird zweckmässig unter alkalischen Bedingungen, z. B. mittels wässrig-alkoholischem Alkalihydroxid, z. B. wässrig-methanolischem Kaliumhydroxyd, vorgenommen. Eine in einer Seitenkette $R^1$ oder $R^{11}$ enthaltene Doppelbindung kann z. B. in Gegenwart eines Katalysators, wie Palladium auf Kohle, in einem Lösungsmittel, wie einem niederen Alkanol, z. B. Aethanol, zur Einfachbindung hydriert werden. Ein Hydroxyrest $R^{11}$ kann in an sich bekannter Weise veräthert werden, z. B. durch Umsetzung mit einem dem Aetherrest entsprechenden Mesylat oder Halogenid und in Gegenwart einer Base wie Kaliumhydroxyd in einem Lösungsmittel wie einem niederen Alkanol, z. B. n-Propanol, oder in Gegenwart von Kalium-t-butylat in einem Lösungsmittel, wie DMSO.

Eine gegebenenfalls mono- oder di-niederalkylierte Carbamoylgruppe $R^1$ oder $R^{11}$ kann durch Reduktion, z. B. mit komplexen Metallhydriden wie $LiAlH_4$, zur entsprechenden Aminomethylgruppe reduziert werden. In analoger Weise kann eine nieder-Alkoxycarbonylgruppe zur Hydroxymethylgruppe reduziert werden.

Die Alkanoylierung oder Phenalkanoylierung der in γ-Stellung zum N-Atom eines Diols der Formel I oder eines alkohols der Formel V-1 vorhandenen Hydroxygruppen kann in an sich bekannter Weise durchgeführt werden, z. B. mittels der entsprechenden Carbonsäure oder eines entsprechenden Säurehalogenids in Gegenwart einer starken Säure, wie Salzsäure.

Die Verbindungen der Formeln VI-X können in an sich bekannter Weise hergestellt werden, z. B. die Verbindungen der Formel IX durch Umsetzung einer Säure der Formel $X^1$—C(H,OH)—COOH mit einem Amin der Formel IV.

Bevorzugte Verbindungen der Formeln I und V-1 sind diejenigen, worin der in einer Phenylgruppe Z enthaltene Substituent $R^{11}$ Hydroxy, nieder-Alkanoyloxy, Sulfamoyl oder eine Gruppe $R^1$, —O—$(CH_2)_q$—OH oder —O—$(CH_2)_q$—O—$(CH_2)_t$—$R^5$ und $R^5$ Wasserstoff, nieder-Alkyl oder Phenyl sind.

Bevorzugte Verbindungen der Formeln I und V-1 sind ferner diejenigen, worin R Wasserstoff ist; diejenigen worin $X^1$ Phenoxymethyl und $X^2$ Phenoxymethyl oder Phenyl ist, insbesondere solche, worin das an einen Phenoxymethylrest $X^1$ oder $X^2$ gebundene C-Atom die S-Konfiguration, bzw. das an einem Phenylrest $X^2$ gebundene C-Atom die R-Konfiguration hat.

Bevorzugte Verbindungen der Formeln I und V-1 sind weiter diejenigen, worin Y Methyl ist, insbesondere solche, worin das an einen Methylrest Y gebundene C-Atom die R-Konfiguration hat.

Bevorzugte Verbindungen der Formeln I und V-1 sind weiter diejenigen, worin Z durch Carbamoyl, Methoxycarbonyl oder 2-(Aethoxy oder Methoxy)-carbonyl-1-methylvinyl substituiertes Phenyl oder Thienyl ist.

Bevorzugt sind weiter Verbindungen der Formeln I und V-1 worin Z durch 6-Hydroxyhexoxy oder 2-Phenäthoxy-2-äthoxy und Verbindungen der Formel V-1, worin Z durch (Aethoxy oder Methoxy)-carbonylmethoxy substituiertes Phenyl ist.

Bevorzugte Verbindungen der Formel V-1 sind weiter diejenigen, worin Z p-Hydroxyphenyl ist.

Besonders bevorzugte Verbindungen der Formeln I und V-I sind diejenigen, worin R Wasserstoff, $X^1$ Phenoxymethyl, $X^2$ Phenoxymethyl oder Phenyl, Y Methyl und Z durch Carbamoyl, Methoxycarbonyl oder 2-(Aethoxy oder Methoxy)-carbonyl-1-methylvinyl substituiertes Phenyl oder Thienyl, oder durch 6-Hydroxyhexyoxy, 2-Phenäthoxy-2-äthoxy ist oder Verbindungen der Formel V-1, worin Z (Aethoxy oder Methoxy)-carbonylmethoxy substituiertes Phenyl ist.

Besonders bevorzugte Verbindungen der Formel V-1 sind ferner solche, worin R Wasserstoff, $X^1$ Phenoxymethyl, Y Methyl und Z p-Hydroxyphenyl ist.

Besonders bevorzugte Verbindungen der Formeln I und V-1 sind weiter solche, worin das an einem Methylrest Y gebundene C-Atom die R-Konfiguration, das an einem Phenoxymethylrest $X^1$ oder $X^2$ gebundene C-Atom die S-Konfiguration und das an einem Phenylrest $X^2$ gebundene C-Atom die R-Konfiguration haben.

Beispiele von bevorzugten Verbindungen der Formeln I sind

das 1,1'-[[(R)-α-Methyl p-[2-(phenäthoxy) äthoxy]-phenäthyl] imino-bis] (S)-3-phenoxy-2-propanol], sowie
der (E)-p[(R)-2-[Bis [(RS)-2-hydroxy-3-phenoxypropyl] amino] propyl]-β-methyl-zimtsäuremethylester,
der p-[(R)-2-[Bis[(RS)-2-hydroxy-3-phenoxypropyl]-amino] propyl]benzoesäuremethylester und
das 6-[p-[(R)-[Bis [(S)-2-hydroxy-3-phenoxypropyl]-amino] propyl] phenoxy-1-hexanol.

Beispiele von bevorzugten Verbindungen der Formel V-1 sind
das (S)-1-[[(R)-α-Methyl-p-[2-(äthoxy) äthoxy] phenäthyl] amino]-3-phenoxypropanol, sowie
der 2-[p-[2-[[(RS)-2-Hydroxy-3-phenoxypropyl] amino]-propyl] phenoxy] essigsäuremethylester,
der (E)-p-[(R)-2-[[(RS)-2-Hydroxy-3-phenoxypropyl] amino] propyl]-β-methyl-zimtsäuremethylester,
das p-[(R)-3-[[(RS)-2-Hydroxy-3-phenoxypropyl] amino] butyl] benzamid,
der p-[(R)-2-[[(RS)-2-Hydroxy-3-phenoxypropyl] amino] propyl] benzoesäuremethylester,
das p-[(R)-2-[[(S)-2-Hydroxy-3-phenoxypropyl] amino] propyl] phenol und
das 6-[p-[(R)-2-[[(S)-2-Hydroxy-3-phenoxypropyl] amino] propyl] phenoxy] hexanol.

Die Amine der Formeln I und V-1, sowie die physiologisch verträglichen Salze davon können als Wirkstoffe in pharmazeutischen Präparaten zur Behandlung der Fettsucht und/oder des Diabetes mellitus, insbesondere des obesen Erwachsenendiabetikers verwendet werden. Im Tierexperiment wurde auf Verabreichung der obigen Verbindungen ein gesteigerter Katabolismus, vor allem der Fette, beobachtet. Weiterhin wurde beobachtet, dass die Verbindungen die Bildung von braunem Fettgewebe bei Ratten und obes-hyperglykämischen Mäusen stimulieren. Bekanntlich wird Defekten des braunen Fettgewebes eine wesentliche Rolle bei der Entstehung der Fettsucht zugeschrieben. An obes-hyperglykämischen Mäusen haben die Verbindungen einen ausgeprägten antidiabetischen Effekt, indem sie hypoglykämisch wirken und die Glykosurie vermindern. Diese Verbindungen zeigen nur eine geringe Wirkung auf Herztätigkeit und Kreislauf. Die Dosierung kann in Abhängigkeit von der Wirkungsstärke der einzelnen Verbindungen und den individuellen Bedürfnissen des Patienten 0,5-1 000 mg, vorzugsweise 2-200 mg pro Tag für einen Erwachsenen betragen, wobei die Dosis als Einzeldosis oder in mehreren Dosen über den Tag verteilt verabreicht werden kann.

Ferner konnte mit den obigen Verbindungen im Tierexperiment eine Erhöhung des Körper-Proteingehaltes und eine Erniedrigung des Fettgehalts nachgewiesen werden. Diese Verbindungen führen demnach zu einer Erhöhung der mageren Körpermasse auf Kosten von Fett. Daher können sie zunächst in der Humanmedizin zur Behandlung von Zuständen, die mit erhöhtem Proteinabbau verbunden sind, z. B. bei Rekonvaleszenz nach Operation, verwendet werden. Dabei liegen die Verabreichungsdosen im gleichen Rahmen wie bei der Behandlung der Fettsucht und/oder des Diabetes mellitus.

Die obigen Verbindungen können auch in der Ernährung von Masttieren, wie Rindern, Schweinen, Schafen und Geflügel, Verwendung finden. Dabei können die Verabreichungsdosen und Verabreichungsformen die gleichen sein wie für Vitamine. Diese Verbindungen können auch als Futterzusatz in Dosen von 0,01-100 mg/kg je nach Substanz, Tierart und Alter eingesetzt werden.

Die pharmazeutischen Präparate enthalten den Wirkstoff zusammen mit einem verträglichen pharmazeutischen, organischen oder anorganischen Trägermaterial, wie z. B. Wasser, Gelatine, Gummi arabicum, Lactose, Stärke, Magnesiumstearat, Talk, pflanzliche Oele, Polyalkylenglykole, Vaseline und dergleichen. Die pharmazeutischen Präparate werden vorzugsweise oral, z. B. in Form von Tabletten, Kapseln, Pillen, Pulver, Granulaten, Lösungen, Sirupen, suspensionen oder Elixiren verabreicht. Die Verabreichung kann aber auch parenteral, z. B. in Form von sterilen Lösungen, Suspensionen oder Emulsionen, erfolgen. Die pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die Aktivität der obigen Verbindungen wird aus den nachstehenden Versuchsresultaten deutlich :

1) Wirkung auf den Sauerstoffverbrauch

Männliche Albinoratten im Gewicht von 160-180 g wurden nach 24 Stunden Fasten in Stoffwechselkäfige gesetzt. Die Käfige wurden mit konstant 6 Liter Raumluft/Minute, die bei einem Taupunkt von 11 °C äquilibriert wurde, belüftet. Von der Abluft wurden nach erneuten Aequilibrierung während Perioden von jeweils 14 Minuten Proben gesammelt und der Sauerstoff- und $CO_2$-Gehalt wurde analysiert. Nach einer Anpassungszeit von 4 Stunden erhielten die in Gruppen zu 6 aufgeteilten Tiere entweder Placebo (5 % Gummi arabicum) oder die Testsubstanz (suspendiert in 5 % Gummi arabicum) per os. Danach wurden 12 Stunden lang die Bestimmungen durchgeführt. In Tabelle I ist der Prozentsatz des gemittelten Sauerstoffverbrauchs nach Medikation während der ersten 3 Stunden und der gesamten Versuchsdauer (12 Stunden) vom Sauerstoffverbrauch der Anpassungsperiode angegeben, wobei entsprechende Korrekturen für Aenderungen in der Placebo-Gruppe berücksichtigt wurden.

Tabelle I

| Verbindung hergestellt in Beispiel No. | Dosis µM/kg | $O_2$-Verbrauch % vom Wert der Vorperiode 1.-3.Stunde | 1.-12. Stunde |
|---|---|---|---|
| 1 | 10 | 133 | 125 |
| 2 | 10 | 122 | 114 |
| 3 | 10 | 132 | 110 |
| 4 | 10 | 121 | 111 |
| 5 | 100 | 151 | 118 |
| 6 | 10 | 113 | 110 |
| 7 | 30 | 144 | 121 |
| 9 | 10 | 138 | 110 |
| 10a) | 30 | 143 | 114 |
| 10b) | 3 | 126 | 107 |
| 10c) | 3 | 130 | 110 |
| 10d) | 10 | 132 | 110 |
| 10e) | 10 | 176 | 149 |
| 10f) | 30 | 150 | 112 |
| 10g) | 10 | 126 | 112 |
| 11 | 3 | 127 | 112 |
| 12a) | 10 | 145 | 130 |
| 12b) | 10 | 148 | 137 |
| 13a) | 30 | 123 | 115 |
| 13b) | 30 | 136 | 113 |
| 14a) | 3 | 134 | 110 |
| 14b) | 10 | 121 | 111 |
| 15a) | 3 | 140 | 118 |
| 16a) | 10 | 136 | 113 |
| 16b) | 100 | 145 | 120 |
| 17a) | 1 | 139 | 118 |
| 17b) | 3 | 136 | 120 |
| 18a) | 1 | 155 | 130 |
| 18b) | 3 | 135 | 120 |

Tabelle I (Fortsetzung)

| Verbindung hergestellt in Beispiel No. | Dosis µM/kg | $O_2$-Verbrauch % vom Wert der Vorperiode 1.-3. Stunde | 1.-12. Stunde |
|---|---|---|---|
| 19a) | 30 | 139 | 111 |
| 19b) | 100 | 123 | 107 |
| 20a) | 3 | 164 | 141 |
| 20b) | 3 | 146 | 129 |
| 21a) | 10 | 150 | 121 |
| 21b) | 30 | 136 | 122 |
| 22 | 30 | 156 | 124 |
| 32a | 1 | 131 | 120 |
| 36b | 10 | 165 | 136 |
| 37 | 3 | 156 | 122 |
| 38 | 30 | 173 | 146 |
| 41 | 1 | 170 | 124 |
| 42 | 3 | 166 | 127 |

2) Katabole Wirkung auf die Lipide

Gruppen von 4 männlichen Albinoratten im Gewicht von 320-360 g wurden ohne Zugang zu Futter in Stoffwechselkäfigen gehalten. Sauerstoffverbrauch und $CO_2$-Produktion wurde während 12 Stunden gemessen. Nach 4 Stunden erhielten die Tiere Placebo (5 % Gummi arabicum) oder die Testsubstanz (suspendiert in Gummi arabicum) per os. In der Tabelle II ist die gemittelte Abnahme des respiratorischen Quotienten ($CO_2/CO_2$) während 8 Stunden nach Verabreichung der Testsubstanz im Vergleich zu den letzten 3 Stunden vor Verabreichung der Testsubstanz angegeben. In den Placebo-Gruppen aufgetretene Aenderungen wurden bei der Berechnung berücksichtigt.

Tabelle II

| Verbindung hergestellt in Beispiel No. | Dosis µM/kg | Aenderung des respiratorischen Quotienten |
|---|---|---|
| 10b) | 10 | -0,037 |
| 10e) | 10 | -0,041 |
| 14a) | 30 | -0,072 |
| 17a) | 3 | -0,038 |

3) Wirkung auf Harn- und Blutglucose und die Bildung von braunem Fettgewebe

Weibliche hyperglykämische Fettmäuse wurden an eine auf 3 g/Tag/Tier begrenzte Futtermenge angepasst. Die Testverbindungen (suspendiert in 5 % Gummi arabicum) oder Placebo (5 % Gummi arabicum) wurden während 15 Tagen zweimal täglich oral verabreicht. Harn wurde während 6 Tagen wöchentlich gesammelt und Harnglucose bestimmt. Blutglucose und das Gewicht des interskapulären braunen Fettgewebes wurden am Versuchsende bestimmt.

Die Versuchsresultate sind in Tabelle III als Prozentsätze der Kontrollwerte angegeben.

Tabelle III

| Verbindung hergestellt in Beisp.No. | Dosis µM/kg pro Tag | Harnglucose 1.Woche/2.Woche | | Blut-glucose | braunes Fettgewebe |
|---|---|---|---|---|---|
| 14a) | 60 | 89% | 31% | 54% | 237% |
| 17b) | 60 | 5% | 5% | 46% | 184% |

Die in den folgenden Beispielen verwendeten Ausgangsmaterialien, insbesondere die Amine der Formel V, worin $X^4$ nieder-Alkyl oder gegebenenfalls durch Fluor, Chlor, Trifluormethyl oder nieder-Alkoxy monosubstituiertes Phenyl ist, und die Amine der Formel IV, sind bekannt bzw. können in an sich bekannter Weise, z. B. wie nachstehend oder in den europäischen Patentanmeldungen 6 735, 21 636 und 94 595 beschrieben, hergestellt werden.

Zur Herstellung des Ausgangsamins der Beispiele 1 und 10$^e$ wurden (RS)-4-(2-Thienyl)-2-butanol, Acetylchlorid und Aluminiumchlorid in Methylenchlorid zum (RS)-3-(5-Acetyl-2-thienyl)-1-methylpropylacetat umgesetzt. Mit NaOH in Methanol wurde dieses zum (RS)-5-(3-Hydroxybutyl)-2-thienylmethylketon verseift. Dieses reargierte in Aethanol in Gegenwart von Natriumäthanolat mit Triäthylphosphonoacetat zum (E)-5-[(RS)-3-Hydroxybutyl]-β-methyl-2-thiophenacrylsäureäthylester. Umsetzung mit p-Toluolsulfochlorid und anschliessende Behandlung mit Natriumazid gab (E)-5-[(RS)-3-Azidobutyl]-β-methyl-2-thiophenacrylsäureäthylester. Daraus wurde durch Reduktion mit Triphenylphosphin und anschliessende Hydrolyse der (E)-5-[(RS)-3-Aminobutyl]-β-methyl-2-thiophenacrylsäureäthylester erhalten, $\varepsilon_{320}$ = 17 465.

Zur Herstellung des Ausgangsamins des Beispiels 5 wurden 2-(2-Thienyl) äthyl-p-toluolsulfonat, Acetylchlorid und Aluminiumchlorid in Methylenchlorid zum 2-[(5-Acetyl-2-thienyl) äthyl]-p-toluolsulfonat umgesetzt (Smp. 111-112°, aus Aethanol). Dieses wurde mit Natriumazid in DMSO in das 5-(2-Azidoäthyl)-2-thienylmethylketon übergeführt. Oxidation mit Natriumhypobromit lieferte die 5-(2-Azidoäthyl)-2-thiophencarbonsäure vom Smp. 53-55°, die mit Thionylchlorid das entsprechende Säurechlorid ergab, welches mit Ammoniak zum 5-(2-Azidoäthyl)-2-thiophencarbonsäureamid umgesetzt wurde (Smp. 104-105°, aus Aethanol). Reaktion mit Triphenylphosphin und Hydrolyse ergaben das 5-(2-Aminoäthyl)-2-thiophencarbonsäureamid, Smp. 134-136° (aus Acetonitril).

Zur Herstellung des Ausgangsamins der Beispiele 6 und 10 g wurden 5-[(RS)-2-Hydroxypropyl]-2-thienylmethylketon und Triäthylphosphonoacetat in Aethanol in Gegenwart von Natriumäthylat zum (E)-5-[(RS)-2-Hydroxypropyl]-β-methyl-2-thiophenacrylsäureäthylester umgesetzt. Mit p-Toluolsulfochlorid wurde daraus der (E)-β-Methyl-5-[(RS)-2-[(p-toluolsulfonyl) oxy] propyl]-2-thiophenacrylsäureäthylester erhalten (Smp. 121°, aus Methylenchlorid-Alkohol). Reaktion mit Natriumazid in DMSO gab den (E)-5-[(RS)-2-Azidopropyl]-β-methyl-2-thiophenacrylsäureäthylester. Reduktion mit Triphenylphosphin und Hydrolyse führte zum (E)-5-[(RS)-2-Aminopropyl]-β-methyl-2-thiophenacrylsäureäthylester, $\varepsilon_{320}$ = 17 970.

Zur Herstellung des Ausgangsamins des Beispiels 7 wurde 4-(5-Acetyl-2-thienyl)-2-butanon mit Aethylenglykol, o-Ameisensäuretriäthylester und p-Toluolsulfonsäure in Methylenchlorid selektiv zum Methyl-5-[2-(2-methyl-1,3-dioxolan 2-yl) äthyl]-2-thienylketon umgesetzt. Oxidation mit natriumhypobromit und anschliessende Hydrolyse gab die 5-(3-Oxobutyl)-2-thiophencarbonsäure. Mit $NaBH_4$ wurde daraus die 5-(3-Hydroxybutyl)-2-thiophencarbonsäure erhalten, welche in Dimethylacetamid mit Methyljodid und Natriumbicarbonat in den Methylester übergeführt wurde. Behandlung mit p-Toluolsulfochlorid in Pyridin und Reaktion mit Natriumazid in DMSO gab den 5-(3-Azidobutyl)-2-thiophencarbonsäuremethylester, aus welchem durch Verseifung die entsprechende Säure erhalten wurde. Mit Thionylchlorid wurde deren Säurechlorid hergestellt, aus dem mit konz. Ammoniak in Diäthyläther das 5-(3-Azidobutyl)-2-thiophencarbonsäureamid erhalten wurde. Reduktion der Azidogruppe mit Triphenylphosphin und anschliessende Hydrolyse gab das (R,S)-5-(3-Aminobutyl)-2-thiophencarbonsäureamid, Smp. 65-75°, $\varepsilon_{256}$ = 7 780, $\varepsilon_{275}$ = 9 900.

Zur Herstellung des Ausgangsamins des Beispiels 8 wird (RS)-5-(3-Hydroxybutyl)-2-thienylmethylketon mit p-Toluolsulfochlorid zum (RS)-3-(5-Acetyl-2-thienyl)-1-methylpropyl-p-toluolsulfonat, Smp. 61-63° umgesetzt. Mit Natriumazid erhält man daraus das Methyl-(RS)-5-(3-azidobutyl)-2-thienylketon, welches katalytisch zum 2-Acetyl-5-[(RS)-3-aminobutyl] thiophen hydriert wird.

Zur Herstellung des Ausgangsamins des Beispiels 9 wurde 2-(p-Toluolsulfonyloxy)-propylthiophen mit Acetylchlorid und Aluminiumtrichlorid in Methylenchlorid zum 5-Acetyl-2-(p-toluolsulfonyloxy)-propylthiophen umgesetzt. Mit Natriumazid in DMSO wurde daraus das 5-(3-Azidopropyl)-2-thienylmethylketon erhalten. Oxidation mit Natriumhypobromit gab die 5-(3-Azidopropyl)-2-thiophencarbonsäure, Smp. 71-72°. Umsetzen dieser Säure mit Thionylchlorid und anschliessende Behandlung mit konz. Ammoniak führte zum 5-(3-Azidopropyl)-2-thiophencarbonsäureamid, Smp. 85-87°. Daraus erhält man nach Behandlung mit Triphenylphosphin und Hydrolyse das 5-(3-Aminopropyl)-2-thiophencarbonsäureamid, Smp. 143,5-144° (aus Wasser).

Zur Herstellung des Ausgangsmaterials des Beispiels 10a wurde p-(2-Bromäthyl)-acetophenon mit Natriumazid in DMSO zum p-(2-Azidoäthyl) acetophenon umgesetzt. Oxidation mit Natriumhypobromit gab die p-(2-Azidoäthyl) benzoesäure (Smp. 130-131°, aus Aceton-Hexan), die mit Thionylchlorid in das entsprechende Säurechlorid und anschliessend mit Ammoniak ins p-(2-Azidoäthyl) benzamid überging. Behandlung milt Triphenylphosphin und Hydrolyse gab das p-(2-Aminoäthyl) benzamid, Smp. 132-133° (aus Aethanol).

Zur Herstellung des Ausgangsamins des Beispiels 10b, des 5-(3-Aminopropyl)-2-thiophencarbonsäure-methylesters, wird die 5-(3-Azidopropyl)-2-thiophencarbonsäure mit Methyljodid verestert und der dabei erhaltene 5-(3-Azidopropyl)-2-thiophencarbonsäuremethylester katalytisch hydriert.

Zur Herstellung des Ausgangsamins des Beispiels 10f wurden α-Methyl-2-thiophenäthanol, Acetylchlorid und Aluminiumchlorid in Methylenchlorid zum (RS)-2-(5-Acetyl-2-thienyl)-1-methyläthylacetat umgesetzt. Mit Natronlauge in Methanol wurde dieses zum 5-[(RS)-2-Hydroxypropyl]-2-thienylmethylketon verseift und anschliessend mit p-Toluolsulfochlorid zum (RS)-2-(5-Acetyl-2-thienyl)-1-methyläthyl-p-toluolsulfonat umgesetzt, Smp. 101-103°. Mit Natriumazid in DMSO wurde daraus das 5-[(RS)-2-Azidopropyl]-2-thienylmethylketon erhalten, welches mit Brom in Natronlauge zur 5-[(RS)-2-Azidopropyl]-2-thiophencarbonsäure oxidiert wurde. Mit $SOCl_2$ wurde daraus das entsprechende Säurechlorid, und aus diesem mit Ammoniak das 5-[(RS)-2-Azidopropyl]-2-thiophencarbonsäureamid hergestellt (Smp. 79-80 °C, aus Diäthyläther). Behandlung mit Triphenylphosphin und Hydrolyse gab das 5-[(RS)-2-Aminopropyl]-2-thiophencarbonsäureamid, Smp. 91-92° aus Acetonitril.

Zur Herstellung des Ausgangsamins des Beispiels 24 wurde p-Aminosulfonylbenzaldehyd mit Diäthyl-cyanomethylphosphonat/NaH in Tetrahydrofuran zum 1-Cyano-2-(4-aminosulfonylphenyl)-äthan umgesetzt, welches in Methanol mit Raney-Cobalt als Katalysator zum 3-(4-Aminosulfonylphenyl)-propylamin hydriert wurde.

## Beispiel 1

2 g (E)-5-[(RS)-3-[[(R)-2-Hydroxyphenäthyl] amino]-butyl]-β-methyl-2-thiophenacrylsäureäthylester (Smp. 72°) und 850 mg Phenylglycidäther wurden in 20 ml DMSO auf 90° erwärmt. Nach 20 Stunden wurden nochmals 850 mg Phenylglycidäther zugegeben und weitere 20 Stunden auf 90° erwärmt. Nach dem Abkühlen wurde das Reaktionsgemisch auf Wasser gegossen und mit Methylenchlorid extrahiert. Chromatographie des Rohproduktes gab 1,8 g (E)-5-[(RS)-3-[[(R)-2-Hydroxyphenäthyl]-[(RS)-2-hydroxy-3-phenoxypropyl] amino] butyl]-β-methyl-2-thiophenacrylsäureäthylester, $[\alpha]_D = -27°$ (0,1 % in MeOH), $\varepsilon_{323} = 16170$.

## Beispiel 2

In zu Beispiel 1 analoger Weise wurden aus 1,6 g 5-[(RS)-3-[[(RS)-β-Hydroxy-m-(trifluormethyl) phenäthyl] amino] butyl]-2-thiophencarbonsäureamid (Smp. 165-166°) und 1,25 g Phenylglycidäther 800 mg 5-[(RS)-3-[[(RS)-2-Hydroxy-3-phenoxypropyl]-[(RS)-2-hydroxy-m-(trifluormethyl) phenäthyl] amino] butyl]-2-thiophencarbonsäureamid, $\varepsilon_{271} = \varepsilon_{276} = 12210$, hergestellt.

## Beispiel 3

In Analogie zu Beispiel 1 wurde 5-[(RS)-3-[[(R)-2-Hydroxyphenäthyl]-[(RS)-2-hydroxy-3-phenoxypropyl] amino~ butyl]-2-thiophencarbonsäureamid hergestellt, $[\alpha]_D = -25°$ (0,1 % in MeOH), $\varepsilon_{276} = 11780$.

## Beispiel 4

In Analogie zu beispiel 1 wurde 5-[(RS)-3-[[(R)-β-Hydroxyphenäthyl]-[(RS)-2-hydroxy-3-phenoxypropyl] amino] butyl]-2-thienylmethylketon hergestellt, $[\alpha]_D = -10°$ (0,1 % in MeOH), $\varepsilon_{296} = 11930$.

## Beispiel 5

3,4 g 5-(2-Aminoäthyl)-2-thiophencarbonsäureamid und 2,7 ml 2,3-Epoxypropylphenyläther wurden in 30 ml DMSO 18 Stunden auf 90° erwärmt. Das Reaktionsgemisch wurde auf Wasser gegossen und mit Methylenchlorid extrahiert. Die getrockneten Methylenchloridlösungen wurden eingedampft und der Rückstand mit Aether-Methanol an Kieselgel chromatographiert. Es wurden 1,5 g 5-[2-[Bis-[(RS)-2-hydroxy-3-phenoxypropyl] amino] äthyl]-2-thiophencarbonsäureamid erhalten, $\varepsilon_{277} = 12580$.

## Beispiel 6

In Analogie zu Beispiel 5 wurde aus (E)-5-[(RS)-2-Aminopropyl]-β-methyl-2-thiophenacrylsäureäthylester und 2,3-Epoxypropylphenyläther der (E)-5-[(RS)-2-[Bis-[(RS)-2-hydroxy-3-phenoxypropyl] amino] propyl]-β-methyl-2-thiophenacrylsäureäthylester erhalten, $\varepsilon_{324} = 17260$.

Beispiel 7

In Analogie zu Beispiel 5 wurde aus 2,3-Epoxypropylphenyläther und (RS)-5-(3-Aminobutyl)-2-thiophencarbonsäureamid das 5-[(RS)-3-[Bis-[(RS)-2-hydroxy-3-phenoxypropyl] amino] butyl]-2-thiophencarbonsäureamid hergestellt, $\varepsilon_{270}$ = 12740, $\varepsilon_{276}$ = 13090.

Beispiel 8

In Analogie zu Beispiel 5 wurde aus 2,3-Epoxypropylphenyläther und 2-Acetyl-5-[(RS)-3-aminobutyl] thiophen das 5-[(RS)-3-[Bis-[(RS)-2-hydroxy-3-phenoxypropyl] amino] butyl]-2-thienylmethylketon hergestellt, $\varepsilon_{270}$ = 10770, $\varepsilon_{277}$ = 10220, $\varepsilon_{296}$ = 11860.

Beispiel 9

921 mg 5-(3-Aminopropyl)-2-thiophencarbonsäureamid und 0,68 ml 2,3-Epoxypropylphenyläther wurden in 15 ml DMSO 14 Stunden auf 90° erwärmt. Das Reaktionsgemisch wurde auf Wasser gegossen und mit Methylenchlorid extrahiert. Die getrockneten Methylenchloridlösungen wurden im Vakuum eingedampft und der Rückstand an Kieselgel chromatographiert. Es wurden 800 mg 5-[3-[[(RS)-2-Hydroxy-3-phenoxypropyl] amino] propyl]-2-thiophencarbonsäureamid erhalten, Smp. 129-130°, $\varepsilon_{276}$ = 12130.

Beispiel 10

In Analogie zu Beispiel 9 wurden hergestellt :

a) p-[2-[[(RS)-2-Hydroxy-3-phenoxypropyl] amino] äthyl]benzamid, Smp. 133-135°, $\varepsilon_{222}$ = 16200 ;

b) 5-[3-[[(RS)-2-Hydroxy-3-phenoxypropyl] amino] propyl]-2-thiophencarbonsäuremethylester, Smp. 90-91°, $\varepsilon_{277}$ = 13510 ;

c) 5-[(RS)-3-[[(RS)-2-Hydroxy-3-phenoxypropyl] amino] butyl]-2-thiophencarbonsäureamid, Smp. 126-128°, $\varepsilon_{276}$ = 12310 ;

d) 5-[2-[[(RS)-2-Hydroxy-3-phenoxypropyl] amino] äthyl]-2-thiophencarbonsäureamid, Smp. 115-117°, $\varepsilon_{276}$ = 11930 ;

e) (E)-5-[(RS)-3-[(RS)-2-Hydroxy-3-phenoxypropyl] amino]-butyl]-β-methyl-2-thiophenacrylsäureäthylester, $\varepsilon_{321}$ = 17130 ;

f) 5-[(RS)-2-[[(RS)-2-Hydroxy-3-phenoxypropyl] amino]-propyl]-2-thiophencarbonsäureamid, $\varepsilon_{277}$ = 11370 ;

g) (E)-5-[(RS)-2-[[(RS)-2-Hydroxy-3-phenoxypropyl] amino]-propyl]-β-methyl-2-thiophenacrylsäureäthylester, $\varepsilon_{320}$ = 17390 ;

h) 5-[(RS)-3-[[(RS)-2-Hydroxy-3-phenoxypropyl] amino]-butyl]-2-thienylmethylketon, $\varepsilon_{264}$ = 9850, $\varepsilon_{294}$ = 12100.

Beispiel 11

Eine Mischung von 1,02 g p-[(R)-3-[[(RS)-2-Hydroxy-3-phenoxypropyl] amino] butyl] benzamid und 1,8 g R-Styroloxid in 30 ml DMSO wurde während 70 Stunden auf 100° erwärmt. Die Reaktionslösung wurde am Hochvakuum eingedampft und der Rückstand auf Kieselgel chromatographiert. Mit Chloroform/n-Propanol/25 % $NH_3$ (230 : 20 : 2) wurde 0,8 g reines, amorphes p-[(R)-3-[[(R)-β-Hydroxyphenäthyl] [(RS)-2-hydroxy-3-phenoxypropyl] amino] butyl] benzamid eluiert, $[\alpha]^{20}$ = —60° (c = 0,6 in Methanol), $\varepsilon_{220}$ = 18190, $\varepsilon_{234}$ = 13765.

Beispiel 12

In Analogie zu Beispiel 11 wurden als amorphe Produkte hergestellt :

a) der p-[(R)-2-[[(R)-β-Hydroxyphenäthyl] [(RS)-2-hydroxy-3-phenoxypropyl] amino] propyl] benzoesäuremethylester, $[\alpha]^{20}_D$ = —68° (c = 0,5 in Methanol), $\varepsilon_{220}$ = 17030, $\varepsilon_{237}$ = 13680 ; und

b) der p-[(R)-2-[[(R)-β-Hydroxyphenäthyl] [(RS)-2-hydroxy-3-phenoxypropyl] amino] propyl]-β-methylzimtsäuremethylester, $[\alpha]^{20}$ = —63° (c = 0,5 in Methanol), $\varepsilon_{270}$ = 17310, $e_{277}$ = 17900.

Beispiel 13

Eine Mischung von 3,8 g S-1-Methyl-3-(4-aminocarbonylphenyl) propylamin und 3,60 g 2,3-Epoxypropylphenyläther in 30 ml Aethanol und 20 ml Acetonitril wurde 8 Stunden unter Rückfluss erhitzt. Die Reaktionslösung wurde dann im vakuum eingedampft und der Rückstand auf 250 g Kieselgel chromatographiert. Mit dem Gemisch Chloroform/n-Propanol/25 % $NH_3$ (1000 : 50 : 5) wurden zunächst 2,4 g amorphes p-[(S)-3-Bis-[(RS)-2-Hydroxyphenoxypropyl] amino] butyl] benzamid eluiert, $[\alpha]^{20}_D$ + 29° (c = 0,4 in Methanol), $\varepsilon_{220}$ = 24025, $\varepsilon_{237}$ = 12940.

Mit dem Gemisch Chloroform/n-Propanol/25 % $NH_3$ (100 : 10 : 1) wurden anschliessend 3,5 g reines p-[(S)-3-[[(RS)-2-Hydroxy-3-phenoxypropyl] amino] butyl] benzamid eluiert, Smp. 133-136° (aus Acetonitril), $[\alpha]^{20}_D$ = — 2° (c = 0,8 in Methanol), $\varepsilon_{223}$ = 15510, $\varepsilon_{236}$ = 13820.

Beispiel 14

In Analogie zu Beispiel 13 wurden hergestellt unter Verwendung von R-1-Methyl-3-(4-aminocarbonylphenyl) propylamin :

a) das p-[(R)-3-[[(RS)-2-Hydroxy-3-phenoxypropyl] amino]-butyl] benzamid, Smp. 132-136° (Acetonitril), $[\alpha]^{20}_D$ = + 2° (c = 1,0 in Methanol), $\varepsilon_{222}$ = 15250, $\varepsilon_{236}$ = 13630 ; und

b) das p-[(R)-3-Bis[[(RS)-2-Hydroxy-3-phenoxypropyl]-amino] butyl] benzamid, amorph, $[\alpha]^{20}_D$ = — 29° (c = 0,4 in Methanol), $\varepsilon_{220}$ = 23700, $\varepsilon_{236}$ = 13010.

Beispiel 15

In Analogie zu Beispiel 13 wurden hergestellt unter Verwendung von (S)-2,3-Epoxypropylphenyläther und R-1-Methyl-3-(4-aminocarbonylphenyl) propylamin

a) das p-[(R)-3-[[(S)-2-Hydroxy-3-phenoxypropyl] amino]-butyl] benzamid, Smp. 129-130° (Aethanol), $[\alpha]^{20}_D$ = + 3° (c = 1,0 in Methanol), $\varepsilon_{223}$ = 14990, $\varepsilon_{236}$ = 13290 ; und

b) das p-[(R)-3-Bis[[(S)-2-hydroxy-3-phenoxypropyl] amino]-butyl] benzamid, $[\alpha]^{20}_D$ = — 24° (c = 0,56 in Methanol), $\varepsilon_{221}$ = 22510, $\varepsilon_{237}$ = 12650.

Beispiel 16

In Analogie zu Beispiel 13 wurden hergestellt :

a) das (RS)-p-[3-[(2-Hydroxy-3-phenoxypropyl) amino]-propyl] benzamid, Smp. 121-122° (Aceton), $\varepsilon_{222}$ = 15170, $\varepsilon_{235}$ = 13540 ; und

b) das (RS)-p-3-Bis[(2-hydroxy-3-phenoxypropyl) amino]-propyl] benzamid, amorph, $\varepsilon_{220}$ = 25010, $\varepsilon_{236}$ = 13930.

Beispiel 17

In Analogie zu Beispiel 13 wurden hergestellt unter Verwendung von p-[(R)-2-Aminopropyl]-β-methylzimtsäuremethylester :

a) der (E)-p-[(R)-2-[[(RS)-2-Hydroxy-3-phenoxypropyl]-amino] propyl]-β-methyl-zimtsäuremethylester, Smp. 70-72° (Aceton-Hexan), $[\alpha]^{20}_D$ = — 22° (c = 0,7 in Methanol), $\varepsilon_{271}$ = 19960, $\varepsilon_{276}$ = 20030 ; und

b) der (E)-p-[(R)-2-[Bis[(RS)-2-hydroxy-3-phenoxypropyl]-amino] propyl]-β-methyl-zimtsäuremethylester, amorph, $[\alpha]^{20}_D$ = — 37° (c = 0,27 in Methanol), $\varepsilon_{271}$ = 15570, $\varepsilon_{277}$ = 19910.

Beispiel 18

In Analogie zu Beispiel 13 wurden hergestellt unter Verwendung von (S)-2,3-Epoxypropylphenyläther und p-[(R)-2-Aminopropyl]-β-methyl-zimtsäuremethylester :

a) der p-[(R)-2-[[(S)-2-Hydroxy-3-phenoxypropyl] amino]-propyl]-β-methyl-zimtsäuremethylester, Smp. 63-64° (Aether-Hexan), $[\alpha]^{20}_D$ = — 25° (c = 0,9 in Methanol) ; $\varepsilon_{220}$ = 19950, $\varepsilon_{272}$ = 19125, $\varepsilon_{277}$ = 19125 ; und

b) der p-[(R)-2-[Bis[(S)-2-hydroxy-3-phenoxypropyl] amino]-propyl]-β-methyl-zimtsäuremethylester, amorph, $[\alpha]^{20}_D$ = — 9,4° (c = 0,8 in Methanol), $\varepsilon_{220}$ = 27200, $\varepsilon_{271}$ = 17700, $\varepsilon_{277}$ = 18000.

Beispiel 19

In Analogie zu Beispiel 13 wurden hergestellt unter Verwendung von (S)-2,3-Epoxypropylphenyläther und p-[(R)-2-Aminopropyl] acetophenon :

a) das 4'-[(R)-2-[[(S)-2-Hydroxy-3-phenoxypropyl] amino]-propyl] acetophenon, Smp. 72-78° (Acetonitril), $[\alpha]^{20}_D$ = — 26° (c = 1,0 in Methanol), $\varepsilon_{253}$ = 15280, $\varepsilon_{276}$ = 4700 ; und

b) das 4'-[(R)-2-[Bis[(S)-2-hydroxy-3-phenoxypropyl] amino]-propyl] acetophenon, amorph, $[\alpha]^{20}_D$ = — 24° (c = 1,0 in Methanol), $\varepsilon_{256}$ = 11780, $\varepsilon_{270}$ = 9570, $\varepsilon_{277}$ = 7650.

Beispiel 20

In Analogie zu Beispiel 13 wurden hergestellt unter Verwendung von 2,3-Epoxypropylphenyläther und p-[(R)-2-Aminopropyl] benzoesäuremethylester :

a) der p-[(R)-2-[[(RS)-2-Hydroxy-3-phenoxypropyl] amino]-propyl] benzoesäuremethylester, amorph, $[\alpha]^{20}_D$ = — 23° (c = 0,9 in Methanol), $\varepsilon_{222}$ = 13890, $\varepsilon_{238}$ = 14890 ; und

b) der p-[(R)-2-[Bis[(RS)-2-hydroxy-3-phenoxypropyl]-amino] propyl] benzoesäuremethylester, amorph, $[\alpha]^{20}$ = — 35° (c = 1,0 in Methanol), $\varepsilon_{220}$ = 23480, $\varepsilon_{238}$ = 13190.

Beispiel 21

In Analogie zu Beispiel 13 wurden hergestellt unter Verwendung von 2,3-Epoxypropyl-2'-fluorphenyläther und R-1-Methyl-3-(4-aminocarbonylphenyl) propylamin :

a) das p-[(R)-3-[[(RS)-(o-Fluorphenoxy)-2-hydroxypropyl]-amino] butyl] benzoesäureamid, Smp. 114-116° (Acetonitril), $[\alpha]^{20}_D$ = + 2,4° (c = 1,0 in Methanol), $\varepsilon_{220}$ = 14145, $\varepsilon_{236}$ = 13720 ; und

b) das p-[(R)-3-[Bis[(RS)-(o-fluorphenoxy)-2-hydroxypropyl] amino] butyl] benzamid, amorph, $[\alpha]^{20}_D$ = — 33° (c = 0,6 in Methanol), $\varepsilon_{220}$ = 21670, $\varepsilon_{236}$ = 12860.

Beispiel 22

In Analogie zu Beispiel 13 wurde hergestellt unter Verwendung von 2,3-Epoxypropyl-2'-chorphenyläther und p-[(R)-2-Aminopropyl]-β-methyl-zimtsäuremethylester :

das p-[(R)-2-[[(RS)-3-(o-Chlorphenoxy)-2-hydroxypropyl]-amino] propyl]-β-methyl-zimtsäuremethylester, amorph, $[\alpha]^{20}_D$ = — 19° (c = 1,0 in Methanol), $\varepsilon_{218}$ = 19865, $\varepsilon_{274}$ = 19865, $\varepsilon_{281}$ = 18910.

Beispiel 23

Eine Mischung von 4,0 g Tyramin und 5,25 g Phenylglycidäther in 100 ml DMSO wurde 17 Stunden auf 100° erwärmt. Das Lösungsmittel wurde im Vakuum abgedampft und der Rückstand auf 400 g Kieselgel chromatographiert. Mit Chloroform/n-Propanol/konz. $NH_3$ (1000 : 10 : 1) wurde zuerst das 4,3 g p-[2-[Bis[(RS)-2-hydroxy-3-phenoxypropyl] amino]-äthyl] phenol als amorphe Substanz eluiert, $\varepsilon_{218}$ = 20710. Die weiteren Fraktionen lieferten 4,2 g reines (RS)-p-[2-[(2-Hydroxy-3-phenoxypropyl) amino] äthyl] phenol vom Smp. 122-124°, $\varepsilon_{221}$ = 15310.

Beispiel 24

In Analogie zu beispiel 23 wurden hergestellt unter Verwendung von 3-(4-Aminosulfonylphenyl) propylamin und Phenylglycidäther :

das (RS)-p-[3-[(2-Hydroxy-3-phenoxypropyl) amino] propyl]-benzosulfonamid, Smp. 119-120° (aus Acetonitril), $\varepsilon_{221}$ = 20160 und

das p-[2-[Bis[(RS)-2-Hydroxy-3-phenoxypropyl] amino]-propyl] benzolsulfonamid, amorph, $\varepsilon_{222}$ = 27450.

Beispiel 25

In Analogie zu Beispiel 23 wurde der p-[(R)-2-[[(RS)-3-Phenoxy-2-hydroxypropyl] amino] propyl]-5-phenyl-3,5-dimethylpenta-2,4-diensäuremethylester, amorph, $[\alpha]^{20}_D$ = — 20° (c = 0,5 in Methanol), $\varepsilon_{270}$ = 11860, $\varepsilon_{276}$ = 12850, $\varepsilon_{294}$ = 13610 hergestellt unter Verwendung von Phenylglycidäther und von p-[(R)-2-Aminopropyl]-5-phenyl-3,5-dimethylpenta-2,4-diensäuremethylester.

Beispiel 26

In Analogie zu Beispiel 23 wurde der p-[(R)-2-[[(RS)-3-Phenoxy-2-hydroxypropyl] amino] propyl]-4-phenyl-4-oxo-buttersäuremethylester, amorph, $[\alpha]^{20}_D = -20°$ (c = 0,3 in Methanol), $\varepsilon_{251}$ = 15250 hergestellt unter Verwendung von Phenylglycidäther und von p-[(R)-2-Aminopropyl]-4-phenyl-4-oxobuttersäuremethylester.

Beispiel 27

Eine Lösung von 342 mg p-[(R)-3-[[(RS)-2-Hydroxy-3-phenoxypropyl] amino]-butyl] benzamid in 5 ml Methanol wurde mit 44 mg Salzsäuregas in 1 ml Methanol behandelt. Die Lösung wurde auf 3 ml konzentriert und mit 3 ml Aether versetzt. Nach mehrstündigem Stehen bei 0° wurden die ausgefallenen Kristalle abgenutscht und aus Methanol-Aether umkristallisiert. Das erhaltene p-[(R)-3-[[(RS)-2-Hydroxy-3-phenoxypropyl] amino] butyl] benzamid-hydrochlorid schmolz bei 156-158°, $[\alpha]^{20}_D = +12°$ (c = 0,7 in Methanol), $\varepsilon_{234}$ = 13410, $\varepsilon_{222}$ = 14970.

Analog wurde mit Oxalsäure das Oxalat hergestellt, Smp. 199-200°, $[\alpha]^{20}_D = +8°$ (c = 0,8 in Methanol), $\varepsilon_{222}$ = 15920, $\varepsilon_{235}$ = 13680.

Beispiel 28

Eine Mischung von 1,0 g p-[(R)-3-[[(RS)-2-hydroxy-3-phenoxypropyl] amino] butyl] benzamid, 500 mg $LiAlH_4$ und 100 ml abs. Tetrahydrofuran wurde 2 Stunden unter Rückfluss erhitzt. Man versetzte vorsichtig mit 20 ml warmen Aether und anschliessend mit 2 ml $Na_2SO_4$-Lösung. Der ausgefallene Niederschlag wurde abfiltriert und das Filtrat im Vakuum eingedampft. Der Rückstand wurde in 6 ml Methanol gelöst und mit 250 mg Oxalsäure versetzt. Zur klaren Lösung gab man dann 25 ml Aether und liess im Kühlschrank auskristallisieren. Man erhielt 0,8 g p-[(R)-3-[[(RS)-2-Hydroxy-3-phenoxypropyl] amino] butyl] benzylamin-oxalat vom Smp. 225°, $[\alpha]^{20}_D = +7°$ (c = 0,5 in Wasser), $\varepsilon_{218}$ = 14250.

Beispiel 29

In zu Beispiel 28 analoger Weise wurde hergestellt:

das p-[(R)-3-Bis[[(RS)-2-hydroxy-3-phenoxypropyl]-amino] butyl] benzylamin, amorph, $[\alpha]^{20} = -19°$ (c = 1,0 in Methanol), $\varepsilon_{220}$ = 21010.

Beispiel 30

Zu einer Mischung von 0,5 g $LiAlH_4$ und 50 ml abs. Tetrahydrofuran tropfte man unter Rühren eine Lösung von 1,0 g (E)-p-[(R)-2-[[(RS)-2-hydroxy-3-phenoxypropyl]-amino] propyl]-β-methyl-zimtsäuremethylester in 50 ml THF. Die Mischung wurde dann 90 Minuten zum Rückfluss erwärmt, abgekühlt, vorsichtig mit 20 ml nassem Aether und anschliessend mit 2 ml $Na_2SO_4$-Lösung versetzt. Der Niederschlag wurde abgenutscht und das Filtrat im Vakuum eingedampft. Man erhielt 1,0 g farbloses Oel, das auf 50 g Kieselgel mit Chloroform/n-Propanol/ges. $NH_3$ (1000 : 100 : 5) chromatographiert 650 mg reines, amorphes (RS)-3-[p-[(R)-2-[[(RS)-2-Hydroxy-3-phenoxypropyl] amino] propyl] phenyl]-1-butanol lieferte, $[\alpha]^{20}_D = -20°$ (c = 0,6 in Methanol), $\varepsilon_{218}$ = 16800.

Beispiel 31

0,5 g p-[(R)-3-[[(RS)-2-Hydroxy-3-phenoxypropyl]-amino] butyl] benzamid wurden in 25 ml mit HCl-Gas gesättigter Essigsäure 24 Stunden auf 40° erwärmt. Darauf wurde das Lösungsmittel im Vakuum entfernt und der kristalline Rückstand aus Acetonitril umkristallisiert. Man erhielt das p-[(R)-3-[[(RS)-2-Acetoxy-3-phenoxypropyl] amino] butyl]-benzamid-hydrochlorid vom Smp. 83-85°, $[\alpha]^{20}_D = +10°$ (c = 0,6 in Methanol), $\varepsilon_{222}$ = 15020, $\varepsilon_{234}$ = 13590.

Beispiel 32

Analog zu Beispiel 23 wurden hergestellt:

a) das p-[(R)-2-[[(S)-2-Hydroxy-3-phenoxypropyl] amino]-propyl] phenol, Smp. 107-108°, $[\alpha]^{20}_D = -28°$ (c = 1,0 in Methanol), $\varepsilon_{221}$ = 15810 und

b) das p-[(R)-2-[Bis[(S)-2-hydroxy-3-phenoxypropyl]-amino] propyl] phenol, amorph, $[\alpha]^{20}_D = -5{,}5°$ (c = 1,0 in MeOH), $\varepsilon_{221}$ = 20850.

Beispiel 33

Eine Lösung von 600 mg p-[(R)-2-[[(S)-2-Hydroxy-3-phenoxypropyl] amino] propyl]-β-methyl-zimmtsäuremethylester in 50 ml Aethanol wurde nach Zugabe von 100 mg 10 % Pd/C mit Wasserstoff bei

Normaldruck hydriert, bis ein Aequivalent $H_2$ aufgenommen wurde. Der Katalysator wurde abfiltriert und das Lösungsmittel im Vakuum abgedampft. Man erhielt reines, amorphes (RS)-p-[(R)-2-[[(S)-2-Hydroxy-3-phenoxypropyl] amino] propyl]-β-methyl-hydrozimtsäuremethylester, $[\alpha]^{20}_D = -21°$ (c = 1,0 in Methanol), $\varepsilon_{218} = 17240$.

Beispiel 34

Analog zu Beispiel 33 wurde hergestellt :

der (RS)-p-[(R)-2-[Bis[(RS)-2-hydroxy-3-phenoxypropyl] amino] propyl]-β-methyl-zimtsäuremethylester, amorph, $[\alpha]^{20}_D = -24°$ (c = 1,0 in Methanol) ; $\varepsilon_{219} = 24650$.

Beispiel 35

Ein Gemisch von 1,0 g p-[2-[[(RS)-2-Hydroxy-3-phenoxypropyl] amino] äthyl] phenol, 487 mg Kaliumhydroxid, 1,02 g 1-(2-Phenäthoxy)-2-mesyloxy-äthan und 20 ml n-Propanol wurde 22 Stunden auf 120° erhitzt. Das Reaktionsgemisch wurde auf Eiswasser gegossen und mit Aethylacetat extrahiert. Der organische Extrakt wurde mit Wasser gewaschen, getrocknet und im Vakuum eingedampft. Den Rückstand chromatographierte man auf 80 g Silicagel. Mit Chloroform/n-Propanol/ges. $NH_3$ (500 : 10 : 1) wurden 700 mg reines (RS)-1-[[3-[p-[2-(Phenäthoxy) äthoxy] phenyl] propyl] amino]-3-phenoxy-2-propanol erhalten, Smp. 76-78° (aus Aceton-Hexan), $\varepsilon_{220} = 18760$.

Beispiel 36

In Analogie zu Beispiel 35 wurden hergestellt :

a) das (S)-1-[[(R)-α-Methyl-p-[2-(phenäthoxy) äthoxy]-phenäthyl] amino]-3-phenoxy-2-propanol, amorph, $[\alpha]^{20}_D = -22°$ (c = 0,7 Methanol), $\varepsilon_{222} = 18750$.

b) das 1,1'-[[(R)-α-Methyl-p-[2-(phenäthoxy) äthoxy]-phenäthyl] imino] bis[(S)-3-phenoxy-2-propanol], amorph, $[\alpha]^{20}_D = -5°$ (c = 0,7 in Methanol), $\varepsilon_{217} = 26900$.

Beispiel 37

Zu einer Lösung von 602 mg p-[(R)-2-[[(S)-2-Hydroxy-3-phenoxypropyl] amino] propyl] phenol und 246 mg Kalium-t-butylat in 10 ml DMSO gab man 300 ml 6-Chlorhexanol und rührte die Reaktionsmischung 1 Stunde bei 80°. Das Reaktionsgemisch wurde im Hochvakuum eingedampft und der Rückstand mit Chloroform/n-Propanol/ges. $NH_3$ (1000 : 50 : 3) auf Silicagel chromatographiert. Man erhielt 500 mg reines, amorphes 6-[p-[(R)-2-[[(S)-2-Hydroxy-3-phenoxypropyl]-amino] propyl] phenoxy] hexanol, $[\alpha]^{20}_D = -21°$ (c = 0,9 in Methanol), $\varepsilon_{222} = 17230$.

Beispiel 38

In Analogie zu Beispiel 37 wurde aus p-[(R)-2-[Bis[(S)-2-hydroxy 3-phenoxypropyl] amino] propyl] phenol

das 6-[p-[(R)-[Bis[(S)-2-hydroxy-3-phenoxypropyl] amino]-propyl] phenoxy]-1-hexanol als amorphe Substanz erhalten, $[\alpha]^{20}_D = +8°$ (c = 1,0 in Methanol), $\varepsilon_{221} = 25020$.

Beispiel 39

Zu einer Lösung von 574 mg p-[2-[[(RS)-2-Hydroxy-3-phenoxypropyl] amino] äthyl] phenol und 300 mg Kalium-t-butylat in 10 ml DMSO gab man 525 mg 4-Brombuttersäureäthylester und rührte die Mischung 6 Stunden bei Raumtemperatur unter Argon. Das Lösungsmittel wurde im Vakuum eingedampft und der Rückstand auf 75 g Silicagel chromatographiert. Mit Chloroform/n-Propanol/ges. $NH_3$ (500 : 10 : 1) wurden 600 mg reines, amorphes 4-[p-[2-[[(RS)-2-Hydroxy-3-phenoxypropyl] amino] äthyl] phenoxy] buttersäureäthylester erhalten, $\varepsilon_{221} = 17510$.

Beispiel 40

Analog zu Beispiel 23 wurde ausgehend von (S)-Phenylglycidäther und 6-[p-[(R)-2-Aminopropyl] phenyl]-5-heptencarbonsäuremethylester erhalten :

der 6-[p-[(R)-2-[[(S)-2-Hydroxy-3-phenoxypropyl]amino] propyl] phenyl]-5-heptencarbonsäure-methylester, amorph, $[\alpha]^{20}_D = -21°$ (c = 0,5 in Methanol), $\varepsilon_{248} = 11090$.

Das Ausgangsamin wurde ausgehend von 4-[(R)-2-Acetylaminopropylacetophenon und dem 5-Carboxypentyltriphenyl-phosphoniumbromid über die 6-[p-[(R)-2-Acetylaminopropyl]-phenyl]-5-heptencarbonsäure erhalten.

Beispiel 41

In Analogie zu Beispiel 35 wurde unter Verwendung von 2-Mesyloxyäthoxyäthan erhalten :

das (S)-1-[[(R)-α-Methyl-p-[2-(äthoxy) äthoxy] phenäthyl] amino]-3-phenoxypropanol, amorph, $[\alpha]^{20}_D = -22°$ (c = 0,5 in Methanol), $\varepsilon_{222} = 17670$.

Beispiel 42

In Analogie zu Beispiel 39 wurde unter Verwendung von Bromessigsäuremethylester hergestellt :

der 2-[p-[2-[[(RS)-2-Hydroxy-3-phenoxypropyl] amino]-propyl] phenoxy] essigsäuremethylester, amorph, $[\alpha]^{20}_D = -39°$ (c = 0,8 in Methanol), $\varepsilon_{220} = 18840$.

Beispiel 43

Eine Lösung von 1,0 g p-[2-[Bis[(RS)-2-hydroxy-3-phenoxypropyl] amino] äthyl] phenol, 0,27 ml Benzylbromid und 257 mg Kaliumhydroxid in 30 ml n-Propanol wurde 18 Stunden auf 120° erwärmt. Das Reaktionsgemisch wurde auf Eiswasser gegossen und mit Aethylacetat extrahiert. Das nach Abdampfen des Lösungsmittels erhaltene Rohprodukt wurde auf Silicagel chromatographiert. Die dünnschichtchromatographisch einheitlichen Fraktionen wurden aus Aceton-Hexan umkristallisiert und lieferten reines 1,1'-[[p-(Benzyloxy) phenäthyl] imino] bis[(RS)-3-phenoxy-2-propanol], Smp. 106-110°, $\varepsilon_{219} = 30020$.

Beispiel 44

Eine Mischung von 0,5 g p-[(R)-2-[[(S)-2-Hydroxy-3-phenoxypropyl] amino] propyl] phenol, 185 mg Kaliumhydroxid, 410 mg 1-(2-Chloräthyl)-4-phenyl-piperazin und 15 ml n-Propanol wurde 2,5 Stunden auf 60° erwärmt. Das Reaktionsgemisch wurde auf Eiswasser gegossen und mit Aethylacetat extrahiert. Nach Abdampfen des Lösungsmittels und Chromatographie des Rohprodukts auf Silicagel mit Chloroform/n-Propanol/ges. $NH_3$ (1000 : 10 : 1) wurden 340 mg reines, amorphes (S)-1-[[(R)-α-Methyl-p-[2-(4-phenyl-1-pierazinyl) äthoxy] phenäthyl] amino]-3-phenoxy-2-propanol erhalten, $[\alpha]^{20}_D = -19°$ (c = 0,8 in Methanol), $\varepsilon_{222} = 21890$.

Beispiel 45

Eine Lösung von 685 mg p-[(R)-3-[[(R)-2-Hydroxy-3-phenoxypropyl] amino] butyl] benzamid und 550 mg Phenacetylchlorid in 10 ml mit HCl gesättigtem Dioxan wurde 28 Stunden bei Raumtemperatur gerührt. Zur Aufarbeitung wurde auf Eiswasser gegossen und mit Aether extrahiert. Die saure wässrige Lösung wurde mit Natronlauge versetzt bis pH 10 und dann mit Essigester extrahiert. Das nach Verdampfen des Lösungsmittels erhaltene Rohprodukt wurde auf Silicagel chromatographiert. Mit Chloroform/n-Propanol/ges. $NH_3$ (200 : 10 : 1) wurde reines (RS)-2-[[(R)-3-(p-Carbamoylphenyl)-1-methylpropyl] amino]-1-phenoxymethyl-phenylacetat eluiert, amorph, $[\alpha]^{20}_D = +5°$ (c = 0,5 in Methanol), $\varepsilon_{234} = 13500$.

Beispiel 46

400 mg p-[(R)-2-[[(RS)-2-Hydroxy-3-phenoxypropyl]-amino] propyl] benzoesäuremethylester wurden in 25 ml 5 % methanolischer KOH-Lösung und 2,5 ml Wasser unter Rühren 4 Stunden auf 50° erwärmt. Die Reaktionsmischung wurde abgekühlt, mit Eiswasser versetzt und mit Aether extrahiert. Die wässrige, alkalische Lösung wurde dann mit verdünnter Salzsäure auf pH 2 angesäuert und im Vakuum zur Trockene eingedampft. Durch mehrmaliges Zugeben und Abdampfen von Toluol wurde der feste Rückstand von Wasser befreit. Der im Hochvakuum bei 60° getrocknete Rückstand wurde dann mit 10 ml abs. Aethanol heiss extrahiert, die alkoholische Lösung abfiltriert und im Vakuum eingedampft. Durch nochmaliges Umlösen aus abs. Aethanol wurde das Produkt von HCl befreit. Man erhielt so reines p-[(R)-2-[[(RS)-2-Hydroxy-3-phenoxypropyl] amino] propyl] benzoesäure-hydrochlorid als farbloser Schaum, $[\alpha]^{20}_D = -11°$ (c = 1,0 in Methanol), $\varepsilon_{234} = 17500$.

Beispiel 47

Analog zu Beispiel 29 wurde hergestellt :

das (RS)-1-[[(RS)-3-[5-(Aminomethyl)-2-thienyl]-1-methylpropyl] amino]-3-phenoxy-2-propanol, $\varepsilon_{221} = 11380$, $\varepsilon_{240} = 8600$, $\varepsilon_{270} = 1700$, $\varepsilon_{277} = 1450$.

Beispiel 48

In Analogie zu Beispiel 9 wurde aus 2,3-Epoxypropylphenyläther und 5-(3-Aminopropyl)-N-butyl-2-thiophencarbonsäureamid hergestellt :

Das 5-[3-[(RS)-2-Hydroxy-3-phenoxypropyl] amino]-propyl]-N-butyl-2-thiophencarbonsäureamid, Smp. 110 °C (Acetonitril).

Das Ausgangsmaterial wird aus 5-(3-Aminopropyl)-2-thiophencarbonsäureamid erhalten, indem man die freie Aminogruppe als 2,5-Dimethylpyrrolderivat schützt, die Amidgruppe butyliert und die 2,5-Dimethylpyrrolgruppe wieder abspaltet.

Beispiel 49

In Analogie zu Beispiel 5 wurde aus 5-(3-Aminopropyl)-N-butyl-2-thiophencarboxamid und 2,3-Epoxypropylphenyläther das 5-[3-[Bis-[(RS)-2-hydroxy-3-phenoxypropyl] amino]-propyl]-N-butyl-2-thiophencarbonsäureamid erhalten, $\varepsilon_{271}$ = 13540, $\varepsilon_{277}$ = 13780.

Das Ausgangsmaterial kann wie folgt hergestellt werden :

5-(3-Aminopropyl-2-thiophencarboxamid wird mit Acetonylaceton umgesetzt zu 5-[3-(2,5-Dimethylpyrrol-1-yl)propyl]-2-thiophencarboxamid, Smp. 144-146 °C (vgl. J. Chem. Soc. Chem. Commun. 800 [1982]). Dieses wird mit n-Butylbromid alkyliert zu N-Butyl-5-[3-(2,5-dimethylpyrrol-1-yl) propyl]-2-thiophencarboxamid (vgl. Synthesis 266 [1976]). Letzteres wird mit Hydroxylaminhydrochlorid gespalten zu 5-(3-Aminopropyl)-N-butyl-2-thiophencarboxamid.

Beispiel 50

In Analogie zu Beispiel 9 wurde das (RS)-1-Phenoxy-3-(p-phenoxyphenäthylamino)-2-propanol hergestellt, $\varepsilon_{219}$ = 20510.

Beispiel 51

In Analogie zu Beispiel 5 wurde aus p-[(R)-3-Aminobutyl]-N-butylbenzamid und 2,3-Epoxypropylphenyläther das p-[(R)-3-[Bis[(RS)-2-hydroxy-3-phenoxypropyl] amino] butyl]-N-butylbenzamid hergestellt, $[\alpha]_D$ = — 18° (0,1 % in Methanol).

Das Ausgangsmaterial wurde aus p-[(R)-3-Aminobutyl]-benzamid in Analogie zu dem in Beispiel 49 für die Herstellung des Ausgangsmaterials beschriebenen Syntheseweg hergestellt.

Beispiel 52

In Analogie zu Beispiel 9 wurde aus 2,3-Epoxypropylphenyläther und p-[(R)-3-Aminobutyl]-N-butylbenzamid das p-[(R)-3-[[(RS)-2-Hydroxy-3-phenoxypropyl] amino] butyl]-N-butylbenzamid erhalten. $[\alpha]_D$ = + 5° (0,1 % in Methanol).

Beispiel 53

In Analogie zu Beispiel 5 wurde aus 5-(3-Aminopropyl)-N,N-dibutyl-2-thiophencarbonsäureamid und 2,3-Epoxypropylphenyläther das 5-[3-[Bis-[(RS)-2-hydroxy-3-phenoxypropyl]-amino] propyl]-N,N-dibutyl-2-thiophencarbonsäureamid erhalten, $\varepsilon_{220}$ = 24420, $\varepsilon_{244}$ = 9200, $\varepsilon_{271}$ = 12040, $\varepsilon_{277}$ = 11530.

Das Ausgangsmaterial wurde aus 5-(3-Aminopropyl)-2-thiophencarbonsäureamid in Analogie zu dem in Beispiel 49 für die Herstellung des Ausgangsmaterials beschriebenen Syntheseweg hergestellt.

Beispiel 54

In Analogie zu Beispiel 9 wurde aus 2,3-Epoxypropylphenyläther und 5-(3-Aminopropyl)-N,N-dibutyl-2-thiophencarbonsäureamid das 5-[3-[[(RS)-2-Hydroxy-3-phenoxypropyl] amino] propyl]-N,N-dibutyl-2-thiophencarbonsäureamid erhalten, $\varepsilon_{219}$ = 13150, $\varepsilon_{244}$ = 9260, $\varepsilon_{270}$ = 10240, $\varepsilon_{276}$ = 9960.

Beispiel 55

2,1 g p-[[(R)-3-[(RS)-2-Hydroxy-3-phenoxypropyl] amino]butyl]-N-butylbenzamid wurden in 92 ml THF portionenweise mit 920 mg $LiAlH_4$ versetzt und 4 Stunden am Rückfluss gekocht. Das Reaktionsgemisch wurde mit 25 ml 2N NaOH zersetzt, mit Wasser verdünnt und 3x mit Methylenchlorid extrahiert. Die Methylenchloridlösungen wurden 2x mit Wasser gewaschen, getrocknet und im Vakuum eingedampft. Es wurden 2,06 g (RS)-1-[[(R)-3-[α-(Butylamino)-p-tolyl]-1-methylpropyl] amino]-3-phenoxy-2-propanol erhalten, $[\alpha]_{365}$ = + 6° (0,1 % in MeOH).

Beispiel 56

In Analogie zu Beispiel 55 wurde aus 5-[3-[Bis-[(RS)-2-hydroxy-3-phenoxypropyl] amino] propyl]-N-butyl-2-thiophencarbonsäureamid das 1,1'-[[3-[5-[(Butylamino) methyl]-2-thienyl] propyl] imino] bis[(RS)-3-phenoxy-2-propanol] erhalten, $\varepsilon_{220}$ = 20120, $\varepsilon_{241}$ = 9090, $\varepsilon_{270}$ = 3350, $\varepsilon_{277}$ = 2800.

Beispiel 57

Zu einer Suspension von 760 mg $LiAlH_4$ in 60 ml THF wurde in 20 Minuten eine Lösung von 1,79 g 5-[3-[[(RS)-2-Hydroxy-2-phenoxypropyl] amino] propyl]-N,N-dibutyl-2-thiophencarbonsäureamid in 28 ml THF bei 25° zugetropft und noch 2,5 Stunden bei 25° gerührt. Die Aufarbeitung erfolgte wie im Beispiel 55 und gab 1,73 g (RS)-1-[[3-[5-[(Dibutylamino) methyl]-2-thienyl] propyl] amino]-3-phenoxy-2-propanol, $\varepsilon_{220}$ = 13050, $\varepsilon_{240}$ = 9350, $\varepsilon_{270}$ = 1950, $\varepsilon_{277}$ = 1560.

Beispiel 58

In Analogie zu Beispiel 57 wurde aus 5-[3-[Bis[(RS)-2-hydroxy-3-phenoxypropyl] amino] propyl]-N,N-dibutyl-2-thiophencarbonsäureamid das 1,1'-[[3-[5-[(Dibutylamino) methyl]-2-thienyl] propyl] imino] bis[(RS)-3-phenoxy-2-propanol] erhalten, $\varepsilon_{220}$ = 22680, $\varepsilon_{240}$ = 9400, $\varepsilon_{270}$ = 3640, $\varepsilon_{277}$ = 2910.

Beispiel 59

In Analogie zu Beispiel 5 wurde das 5-[(RS)-2-[Bis-[(RS)-2-hydroxy-3-phenoxypropyl] amino] propyl]-2-thiophencarbonsäureamid hergestellt, $\varepsilon_{220}$ = 32320, $\varepsilon_{271}$ = 11280, $\varepsilon_{277}$ = 11370.

Beispiel 60

In Analogie zu Beispiel 55 wurde das 1,1'-[[(RS)-2-[5-(Aminomethyl)-2-thienyl]-1-methyläthyl]imino]bis[(RS)-3-phenoxy-2-propanol] hergestellt, $\varepsilon_{220}$ = 19200, $\varepsilon_{242}$ = 7770, $\varepsilon_{271}$ = 3750, $\varepsilon_{277}$ = 3070.

Beispiel 61

Es werden in üblicher Weise Tabletten folgender Zusammensetzung hergestellt :

| | |
|---|---|
| Wirkstoff, z. B. E-p-[(R)-2-[Bis[(RS)-2-hydroxy-3-phenoxypropyl]amino]propyl]-β-methyl-zimtsäuremethylester | 250 mg |
| Lactose | 200 mg |
| Maisstärke | 300 mg |
| Maisstärkepaste | 50 mg |
| Calciumstearat | 5 mg |
| Dicalciumphosphat | 45 mg |

**Patentansprüche** (für die Vertragsstaaten : BE, CH, DE, FR, GB, IT, LI, LU, NL, SE)

1. Phenoxypropanolamine der Formel

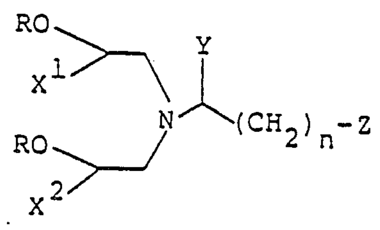

(I)

worin

n die Zahl 1 oder 2,

R Wasserstoff, nieder-Alkanoyl oder Phenyl-nieder-alkanoyl,

$X^1$ gegebenenfalls in ortho-Stellung mono-fluoriertes oder -chloriertes Phenoxymethyl,

$X^2$ nieder-Alkyl, gegebenenfalls in ortho-Stellung mono-fluoriertes oder -chloriertes Phenoxymethyl oder gegebenenfalls durch Fluor, Chlor, Trifluormethyl oder nieder-Alkoxy monosubstituiertes Phenyl,

Y Wasserstoff oder Methyl,

Z eine Gruppe der Formel

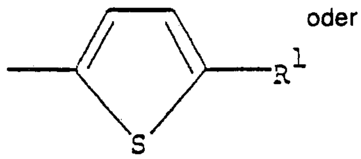

oder

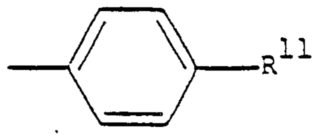

$R^1$ gegebenenfalls N-mono- oder N-di-nieder-alkyliertes Aminomethyl oder ein Rest $-C(O)R^2$,
$-C(R^3) = CH-(CH_2)_m-C(O)R^2$,
$-C(H,R^3)-(CH_2)_{m+1}-C(O)R^2$,
$-C(H,R^3)-(CH_2)_p-OH$ oder
$-C(R^3) = CH-C(CH_3) = CH-COOCH_3$,

$R^{11}$ Hydroxy, nieder-Alkanoyloxy, Sulfamoyl, gegebenenfalls durch Fluor, Chlor, Trifluormethyl, nieder-Alkyl oder nieder-Alkoxy ringsubstituiertes Benzyloxy oder Phenoxy oder eine Gruppe $R^1$,
$-O-(CH_2)_q-OH$,
$-O-(CH_2)_q-O-(CH_2)_t-R^5$ oder

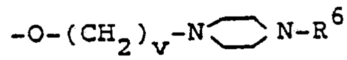

$R^2$ Hydroxy, nieder-Alkyl, nieder-Alkoxy, Dimethylaminoäthoxy, nieder-Alkoxycarbonyläthyl oder gegebenenfalls mono- oder di-niederalkyliertes Amino,

$R^3$ Wasserstoff oder Methyl,

$R^5$ Wasserstoff, nieder-Alkyl oder Phenyl,

$R^6$ nieder-Alkyl oder gegebenenfalls durch Fluor, Chlor, nieder-Alkyl oder nieder-Alkoxy para-substituiertes Phenyl,

m und p ganze Zahlen von 0 bis 6,

v eine ganze Zahl von 2 bis 4,

q und t ganze Zahlen von 1 bis 6 sind,

und physiologisch verträgliche Salze davon.

2. Phenoxypropanolamine der Formel

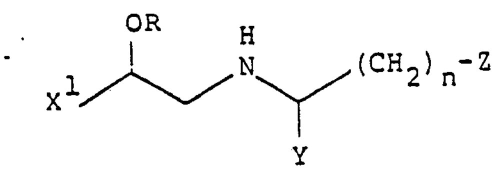

V-1

worin $X^1$, R, Y, Z und n die früher angegebene Bedeutung haben,
und physiologisch verträgliche Salze davon.

3. Verbindungen nach Anspruch 1 oder 2, worin der in einer Phenylgruppe Z enthaltene Substituent $R^{11}$ Hydroxy, nieder-Alkanoyloxy, Sulfamoyl oder eine Gruppe $R^1$, $-O-(CH_2)_q-OH$ oder $-O-(CH_2)_q-O-(CH_2)_t-R^5$ ist, und die restlichen Symbole die gleiche Bedeutung wie in Anspruch 1 oder 2 haben.

4. Verbindungen nach einem der Ansprüche 1-3, worin R Wasserstoff ist.

5. Verbindungen nach einem der Ansprüche 1-4, worin $X^1$ Phenoxymethyl und $X^2$ Phenoxymethyl oder Phenyl ist, insbesondere solche, worin das an einem Phenoxymethylrest $X^1$ oder $X^2$ gebundene C-Atom die S-Konfiguration, bzw. das an einem Phenylrest $X^2$ gebundene C-Atom die R-Konfiguration hat.

6. Verbindungen nach einem der Ansprüche 1-5, worin Y Methyl ist, insbesondere solche, worin das an einem Methylrest Y gebundene C-Atom die R-Konfiguration hat.

7. Verbindungen nach einem der Ansprüche 1-6, worin Z durch Carbamoyl, Methoxycarbonyl oder 2-(Aethoxy oder Methoxy)-carbonyl-1-methylvinyl substituiertes Phenyl oder Thienyl ist.

8. Verbindungen nach einem der Ansprüche 1-6, worin Z durch 6-Hydroxyhexoxy, oder 2-Phenäthoxy-2-äthoxy substituiertes Phenyl sit.

9. Verbindungen nach einem der Ansprüche 2-6, worin Z p-Hydroxyphenyl ist.

10. Verbindungen nach einem der Ansprüche 1-8, worin R Wasserstoff, $X^1$ Phenoxymethyl, $X^2$ Phenoxymethyl oder Phenyl, Y Methyl und Z durch Carbamoyl, Methoxycarbonyl oder 2-(Aethoxy oder Methoxy)-carbonyl-1-methylvinyl substituiertes Phenyl oder Thienyl, oder durch 6-Hydroxyhexoxy oder, 2-Phenäthoxy-2-äthoxy substituiertes Phenyl ist.

11. Verbindungen nach einem der Ansprüche 2-6 und 9, worin R Wasserstoff, $X^1$ Phenoxymethyl, Y Methyl und Z p-Hydroxyphenyl ist.

12. Verbindungen nach einem der Ansprüche 1-11, worin das an einem Methylrest Y gebundene C-Atom die R-Konfiguration, das an einem Phenoxymethylrest $X^1$ oder $X^2$ gebundene C-Atom die S-Konfiguration und das an einem Phenylrest $X^2$ gebundene C-Atom die R-Konfiguration haben.

13. 1,1'-[[(R)-α-Methyl-p-[2-(phenäthoxy)äthoxy]-phenäthyl]imino]bis[(S)-3-phenoxy-2-propanol] und (S)-1-[[(R)-α-Methyl-p-[2-(äthoxy)äthoxy]phenäthyl]amino]-3-phenoxypropanol.

14. (E)-p-[(R)-2-[Bis[(RS)-2-hydroxy-3-phenoxypropyl]amino]propy]-β-methyl-zimtsäuremethylester, p-[(R)-2-[Bis[(RS)-2-hydroxy-3-phenoxypropyl]amino]-propyl]benzoesäuremethylester, 6-[p-(R)-[Bis[(S)-2-hydroxy-3-phenoxypropyl]amino]-propyl]phenoxy-1-hexanol, (E)-p-[(R)-2-[[(RS)-2-Hydroxy-3-phenoxypropyl]-amino]propyl]-β-methyl-zimtsäremethylester, p-[(R)-3-[[(RS)-2-Hydroxy-3-phenoxypropyl]amino]-butyl]benzamid, p-[(R)-2-[[(RS)-2-Hydroxy-3-phenoxypropyl]amino]-propyl]benzoesäuremethylester, p-[(R)-2-[[(S)-2-Hydroxy-3-phenoxypropyl]amino]-propyl]phenol und 6-[p-[(R)-2-[[(S)-2-Hydroxy-3-phenoxypropyl]amino]-propyl]phenoxy]hexanol.

15. Verbindungen nach den Ansprüchen 1-14, sowie Verbindungen der Formel V-1, worin Z durch nieder-Alkoxy oder durch eine Gruppe $-O-(CH_2)_q-COOR^4$ oder $-O-(CH_2)_q-O-(CH_2)_t-R^5$ substituiertes Phenyl ist, worin $R^4$ nieder-Alkyl und $R^5$ durch Chlor, Fluor, Trifluormethyl, nieder-Alkyl oder nieder-Alkoxy para-substituiertes Phenyl ist, als therapeutisch wirksame Mittel, insbesondere als Mittel zur Behandlung der Fettsucht und des Diabetes mellitus bzw. von Zuständen, die mit erhöhtem Proteinabbau verbunden sind.

16. Verbindungen nach den Ansprüchen 1-14, sowie Verbindungen der Formel V-1, worin Z durch nieder-Alkoxy oder durch eine Gruppe $-O-(CH_2)_q-COOR^4$ oder $-O-(CH_2)_q-O-(CH_2)_t-R^5$ substituiertes Phenyl ist, worin $R^4$ nieder-Alkyl und $R^5$ durch Chlor, Fluor, Trifluormethyl, nieder Alkyl oder nieder-Alkoxy para-substituiertes Phenyl ist, als Futterzusatz.

17. Arzneimittel, insbesondere zur Behandlung der Fettsucht und des Diabetes mellitus bzw. von Zuständen, die mit erhöhtem Proteinabbau verbunden sind, enthaltend eine Verbindung nach einem der Ansprüche 1-14 oder eine Verbindung der Formel V-1, worin Z durch nieder-Alkoxy oder durch eine Gruppe $-O-(CH_2)_q-COOR^4$ oder $-O-(CH_2)_q-O-(CH_2)_t-R^5$, substituiertes Phenyl ist, worin $R^4$ nieder-Alkyl und $R^5$ durch Chlor, Fluor, Trifluormethyl, nieder-Alkyl oder nieder-Alkoxy para-substituiertes Phenyl ist.

18. Verwendung von Verbindungen nach den Ansprüchen 1-14 sowie Verbindungen der Formel V-1, worin Z durch nieder-Alkoxy oder durch eine Gruppe $-O-(CH_2)_q-COOR^4$ oder $-O-(CH_2)_q-O-(CH_2)_t-R^5$ substituiertes Phenyl ist, worin $R^4$ nieder-Alkyl und $R^5$ durch Chlor, Fluor, Trifluormethyl, nieder-Alkyl oder nieder-Alkoxy para-substituiertes Phenyl ist, bei der Herstellung von Arzneimitteln zur Behandlung der Fettsucht und des Diabetes mellitus bzw., von Zuständen, die mit erhöhtem Proteinabbau verbunden sind.

**Patentansprüche** (für den Vertragsstaat AT)

1. Verfahren zur Herstellung von Phenoxypropanolaminen der Formel

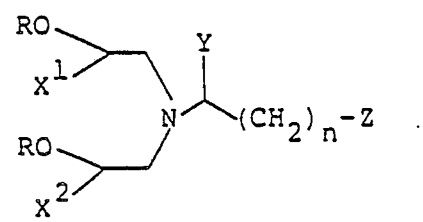

(I)

worin

n die Zahl 1 oder 2,

R Wasserstoff, nieder-Alkanoyl oder Phenyl-nieder-alkanoyl,

$X^1$ gegebenenfalls in ortho-Stellung mono-fluoriertes oder -chloriertes Phenoxymethyl,

$X^2$ nieder-Alkyl, gegebenenfalls in ortho-Stellung mono-fluoriertes oder -chloriertes Phenoxymethyl oder gegebenenfalls durch Fluor, Chlor, Trifluormethyl oder nieder-Alkoxy monosubstituiertes Phenyl,

Y Wasserstoff oder Methyl,

Z eine Gruppe der Formel

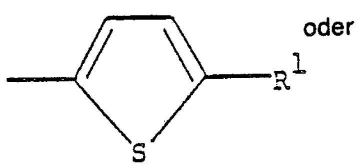

oder

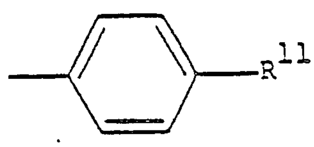

$R^1$ gegebenenfalls N-mono- oder N-di-nieder-alkyliertes Aminomethyl oder ein Rest $-C(O)R^2$,
$-C(R^3)=CH-(CH_2)_m-C(O)R^2$,
$-C(H,R^3)-(CH_2)_{m+1}-C(O)R^2$,
$-C(H,R^3)-(CH_2)_p-OH$ oder
$-C(R^3)=CH-C(CH_3)=CH-COOCH_3$,

$R^{11}$ Hydroxy, nieder-Alkanoyloxy, Sulfamoyl, gegebenenfalls durch Fluor, Chlor, Trifluormethyl, nieder-Alkyl oder nieder-Alkoxy ringsubstituiertes Benzyloxy oder Phenoxy oder eine Gruppe $R^1$, $—O—(CH_2)_q—OH$,

$—O—(CH_2)_q—O—(CH_2)_t—R^5$ oder

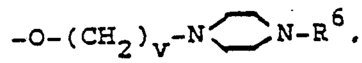

$R^2$ Hydroxy, nieder-Alkyl, nieder-Alkoxy, Dimethylaminoäthoxy, nieder-Alkoxycarbonyläthyl oder gegebenenfalls mono- oder di-niederalkyliertes Amino,

$R^3$ Wasserstoff oder Methyl,

$R^5$ Wasserstoff, nieder-Alkyl oder Phenyl,

$R^6$ nieder-Alkyl oder gegebenenfalls durch Fluor, Chlor, nieder-Alkyl oder nieder-Alkoxy para-substituiertes Phenyl,

m und p ganze Zahlen von 0 bis 6,

v eine ganze Zahl von 2 bis 4,

q und t ganze Zahlen von 1 bis 6 sind,

und von physiologisch verträglichen Salzen davon, dadurch gekennzeichnet, dass man

a) ein Epoxyd der Formel

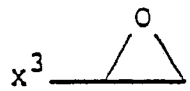
(II)

oder ein β-Ketohalogenid der Formel

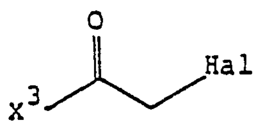
(III)

mit einem primären Amin der Formel

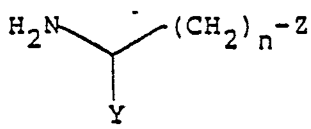
(IV)

oder einem sekundären Amin der Formel

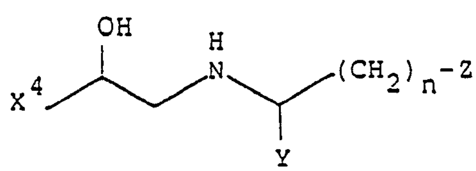
(V)

umsetzt,

wobei in den Formeln II-V n, Y und Z die obige Bedeutung haben, Hal Halogen, eine der Gruppen $X^3$ und $X^4$ eine Gruppe $X^1$ und die andere eine Gruppe $X^2$ ist, wobei ferner $X^3$ eine Gruppe $X^1$ ist, falls man eine Verbindung der Formel II oder III mit einer solchen der Formel IV umsetzt,

und eine in einer erhaltenen Verbindung vorhandene $X^1—C(O)—$ oder $X^2—C(O)$-Gruppe zu einer $X^1—CHOH—$ bzw. $X^2—CHOH$-Gruppe reduziert,

b) gewünschtenfalls einen in einer Gruppe Z einer Verbindung der Formel I enthaltenden reaktionsfähigen Substituenten funktionell abwandelt,

c) gewünschtenfalls die in einem Diol der Formel I vorhandenen Hydroxygruppen alkanoyliert oder phenalkanoyliert, und

d) gewünschtenfalls eine Verbindung der Formel I in ein Salz überführt.

2. Verfahren zur Herstellung von Phenoxypropanolaminen der Formel

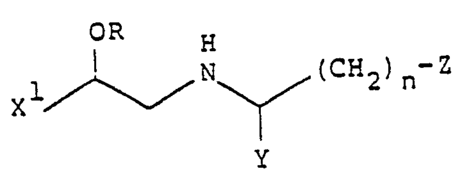
V-1

worin $X^1$, R, Y, Z und n die früher angegebene Bedeutung haben, und von physiologisch verträglichen Salzen davon, dadurch gekennzeichnet, dass man

a) ein Epoxyd der Formel

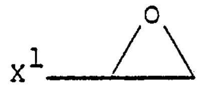

II-1

oder ein β-Ketohalogenid der Formel

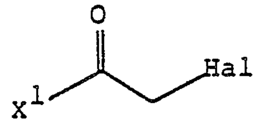

III-1

mit einem Amin der Formel IV umsetzt, und eine in einer erhaltenen Verbindung vorhandene $X^1$—C(O)-Gruppe zur $X^1$—CH(OH)-Gruppe reduziert, oder

b) eine Verbindung einer der Formeln

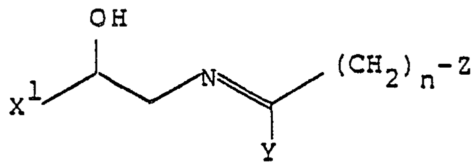

(VI)

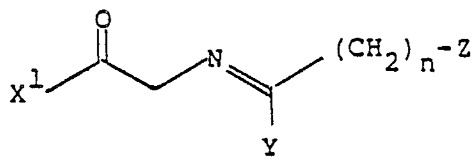

(VII)

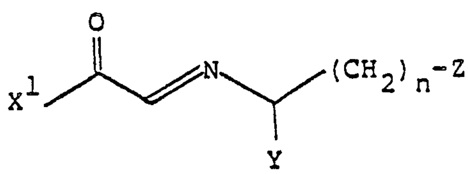

(VIII)

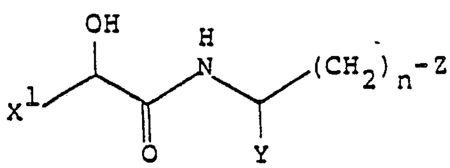

(IX)

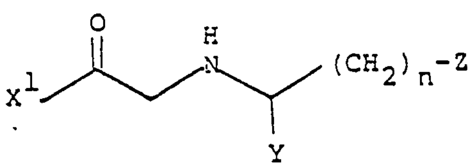

(X)

reduziert,

c) gewünschtenfalls einen in einer Gruppe Z einer Verbindung der Formel V-1 enthaltenen reaktionsfähigen Substituenten funktionell abwandelt,

d) gewünschtenfalls die in einem Alkohol der Formel V-1 vorhandene Hydroxygruppe alkanoyliert oder phenalkanoyliert, und

e) gewünschtenfalls eine Verbindung der Formel V-1 in ein Salz überführt,

wobei in den vorstehenden Formeln die Reste $X^1$, Y, Z und n die früher angegebene Bedeutung haben.

3. Verfahren zur Herstellung von Verbindungen nach Anspruch 1 oder 2, worin der in einer Phenylgruppe Z enthaltene Substituent $R^{11}$ Hydroxy, nieder-Alkanoyloxy, Sulfamoyl oder eine Gruppe

$R^1$, $-O-(CH_2)_q-OH$ oder $-O-(CH_2)_q-O-(CH_2)_t-R^5$ ist, und die restlichen Symbole die gleiche Bedeutung wie in Anspruch 1 oder 2 haben, dadurch gekennzeichnet, dass man entsprechend substituierte Ausgangsmaterialien einsetzt.

4. Verfahren zur Herstellung von Verbindungen nach einem der Ansprüche 1-3, worin R Wasserstoff ist, dadurch gekennzeichnet, dass man entsprechend substituierte Ausgangsmaterialien einsetzt.

5. Verfahren zur Herstellung von Verbindungen nach einem der Ansprüche 1-4, worin $X^1$ Phenoxymethyl und $X^2$ Phenoxymethyl oder Phenyl ist, insbesondere solche, worin das an einem Phenoxymethylrest $X^1$ oder $X^2$ gebundene C-Atom die S-Konfiguration, bzw. das an einem Phenylrest $X^2$ gebundene C-Atom die R-Konfiguration hat, dadurch gekennzeichnet, dass man entsprechend substituierte Ausgangsmaterialien einsetzt.

6. Verfahren zur Herstellung von Verbindungen nach einem der Ansprüche 1-5, worin Y Methyl ist, insbesondere solche, worin das an einem Methylrest Y gebundene C-Atom die R-Konfiguration hat, dadurch gekennzeichnet, dass man entsprechend substituierte Ausgangsmaterialien einsetzt.

7. Verfahren zur Herstellung von Verbindungen nach einem der Ansprüche 1-6, worin Z durch Carbamoyl, Methoxycarbonyl oder 2-(Aethoxy oder Methoxy)-carbonyl-1-methylvinyl substituiertes Phenyl oder Thienyl ist, dadurch gekennzeichnet, dass man entsprechend substituierte Ausgangsmaterialien einsetzt.

8. Verfahren zur Herstellung von Verbindungen nach einem der Ansprüche 1-6, worin Z durch 6-Hydroxyhexoxy oder 2-Phenäthoxy-2-äthoxy substituiertes Phenyl ist, dadurch gekennzeichnet, dass man entsprechend substituierte Ausgangsmaterialien einsetzt.

9. Verfahren zur Herstellung von Verbindungen nach einem der Ansprüche 2-6, worin Z p-Hydroxyphenyl ist, dadurch gekennzeichnet, dass man entsprechen substituierte Ausgangsmaterialien einsetzt.

10. Verfahren zur Herstellung von Verbindungen nach einem der Ansprüche 1-8, worin R Wasserstoff, $X^1$ Phenoxymethyl, $X^2$ Phenoxymethyl oder Phenyl, Y Methyl und Z durch Carbamoyl, Methoxycarbonyl oder 2-(Aethoxy oder Methoxy)-carbonyl-1-methylvinyl substituiertes Phenyl oder Thienyl, oder durch 6-Hydroxyhexoxy oder 2-Phenäthoxy-2-äthoxy substituiertes Phenyl ist, dadurch gekennzeichnet, dass man entsprechend substituierte Ausgangsmaterialien einsetzt.

11. Verfahren zur Herstellung von Verbindungen nach einem der Ansprüche 2-6 und 9, worin R Wasserstoff, $X^1$ Phenoxymethyl, Y Methyl und Z p-Hydroxyphenyl ist, dadurch gekennzeichnet, dass man entsprechend substituierte Ausgangsmaterialien einsetzt.

12. Verfahren zur Herstellung von Verbindungen nach einem der Ansprüche 1-11, worin das an einem Methylrest Y gebundene C-Atom die R-Konfiguration, das an einem Phenoxymethylrest $X^1$ oder $X^2$ gebundene C-Atom die S-Konfiguration und das an einem Phenylrest $X^2$ gebundene C-Atom die R-Konfiguration haben, dadurch gekennzeichnet, dass man entsprechend substituierte Ausgangsmaterialien einsetzt.

13. Verfahren nach Anspruch 1 oder 2 zur Herstellung des 1,1'-[[(R)-α-Methyl -p-[2-(phenäthoxy)äthoxy]phenäthyl]imino]bis[(S)-3-phenoxy-2-propanols] oder des (S)-1-[[(R)-α-Methyl -p-[2-(äthoxy)äthoxy]phenäthyl]amino]-3-phenoxypropanols, dadurch gekennzeichnet, dass man entsprechend substituierte Ausgangsmaterialien einsetzt.

14. Verfahren nach Anspruch 1 oder 2 zur Herstellung des (E)-p-[(R)-2-[Bis-[(RS)-2-hydroxy-3-phenoxypropyl]amino]propyl]-β-methylzimtsäuremethylesters,
p-[(R)-2-[Bis[(RS)-2-hydroxy-3-phenoxypropyl]amino]-propyl]benzoesäuremethylesters,
6-[p-(R)-[Bis[(S)-2-hydroxy-3-phenoxypropyl]amino]-propyl]phenoxy-1-hexanols,
(E)-p-[(R)-2-[[(RS)-2-Hydroxy-3-phenoxypropyl]-amino]propyl]-β-methyl-zimtsäuremethylesters,
p-[(R)-3-[[(RS)-2-Hydroxy-3-phenoxypropyl]amino]-butyl]benzamids,
p-[(R)-2-[[(RS)-2-Hydroxy-3-phenoxypropyl]amino]-propyl]benzoesäuremethylesters,
p-[(R)-2-[[(S)-2-Hydroxy-3-phenoxypropyl]amino]-propyl]phenols oder
des 6-[p-[(R)-2-[[(S)-2-Hydroxy-3-phenoxypropyl]-amino]propyl]phenoxy]hexanols,
dadurch gekennzeichnet, dass man entsprechend substituierte Ausgangsmaterialien einsetzt.

**Claims** (for the Contracting States : BE, CH, DE, FR, GB, IT, LI, LU, NL, SE)

1. Phenoxypropanolamines of the formula

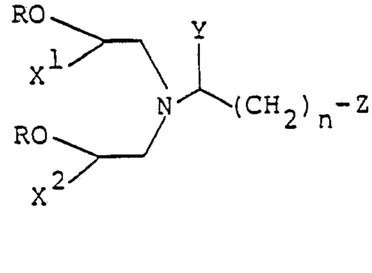

(I)

wherein

n is the number 1 or 2,

R is hydrogen, lower-alkanoyl or phenyl-lower-alkanoyl,

$X^1$ is phenoxymethyl optionally mono-fluorinated or mono-chlorinated in the ortho-position,

$X^2$ is lower-alkyl, phenoxymethyl optionally monofluorinated or mono-chlorinated in the ortho-position or phenyl optionally monosubstituted by fluorine, chlorine, trifluoromethyl or lower-alkoxy,

Y is hydrogen or methyl,

Z is a group of the formula

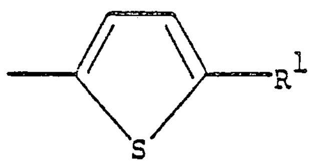

or

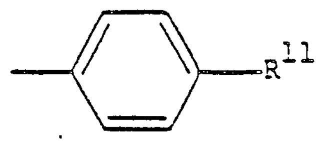

$R^1$ is optionally N-mono-lower-alkylated or N-di-lower-alkylated aminomethyl or a residue $-C(O)R^2$, $-C(R^3)=CH-(CH_2)_m-C(O)R^2$, $-C(H,R^3)-(CH_2)_{m+1}-C(O)R^2$, $-C(H,R^3)-(CH_2)_p-OH$ or $-C(R^3)=CH-C(CH_3)=CH-COOCH_3$,

$R^{11}$ is hydroxy, lower-alkanoyloxy, sulphamoyl, benzyloxy or phenoxy optionally ring-substituted by fluorine, chlorine, trifluoromethyl, lower-alkyl or lower-alkoxy, or a group $R^1$, $-O-(CH_2)_q-CH$, $-O-(CH_2)_q-O-(CH_2)_t-R^5$ or

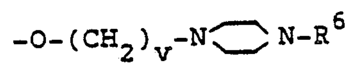

$R^2$ is hydroxy, lower-alkyl, lower-alkoxy, dimethylaminoethoxy, lower-alkoxycarbonylethyl or optionally mono-lower alkylated or di-lower alkylated amino,

$R^3$ is hydrogen or methyl,

$R^5$ is hydrogen, lower-alkyl or phenyl,

$R^6$ is lower-alkyl or phenyl optionally para-substituted by fluorine, chlorine, lower-alkyl or lower-alkoxy,

m and p are whole numbers of 0 to 6,

v is a whole number of 2 to 4,

q and t are whole numbers of 1 to 6,

and physiologically compatible salts thereof.

2. Phenoxypropanolamines of the formula

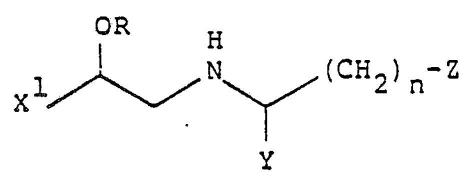

V-1

wherein $X^1$, R, Y, Z and n have the significance given earlier, and physiologically compatible salts thereof.

3. Compounds according to claim 1 or 2, wherein the substituent $R^{11}$ present on a phenyl group Z is hydroxy, lower-alkanoyloxy, sulphamoyl or a group $R^1$, $-O-(CH_2)_q-OH$ or $-O-(CH_2)_q-O-(CH_2)_t-R^5$ and the remaining symbols have the same significance as in claim 1 or 2.

4. Compounds according to any one of claims 1-3, wherein R is hydrogen.

5. Compounds according to any one of claims 1-4, wherein $X^1$ is phenoxymethyl and $X^2$ is phenoxymethyl or phenyl, especially those in which the C-atom bonded to a phenoxymethyl residue $X^1$ or $X^2$ has the S-configuration or the C-atom bonded to a phenyl residue $X^2$ has the R-configuration.

6. Compounds according to any one of claims 1-5, wherein Y is methyl, especially those in which the C-atom bonded to a methyl residue Y has the R-configuration.

7. Compounds according to any one of claims 1-6, wherein Z is phenyl or thienyl substituted by carbamoyl, methoxycarbonyl or 2-(ethoxy or methoxy)-carbonyl-1-methylvinyl.

8. Compounds according to any one of claims 1-6, wherein Z is phenyl substituted by 6-hydroxyhexoxy or 2-phenethoxy-2-ethoxy.

9. Compounds according to any one of claims 2-6, wherein Z is p-hydroxyphenyl.

10. Compounds according to any one of claims 1-8, wherein R is hydrogen, $X^1$ is phenoxymethyl, $X^2$ is phenoxymethyl or phenyl, Y is methyl and Z is phenyl or thienyl substituted by carbamoyl, methoxycarbonyl or 2-(ethoxy or methoxy)-carbonyl-1-methylvinyl, or phenyl substituted by 6-hydroxyhexoxy or 2-phenethoxy-2-ethoxy.

11. Compounds according to any one of claims 2-6 and 9, wherein R is hydrogen, $X^1$ is phenoxymethyl, Y is methyl and Z is p-hydroxyphenyl.

12. Compounds according to any one of claims 1-11, wherein the C-atom bonded to a methyl residue Y has the R-configuration, the C-atom bonded to a phenoxymethyl residue $X^1$ or $X^2$ has the S-configuration and the C-atom bonded to a phenyl residue $X^2$ has the R-configuration.

13. 1,1'-[[(R)-α-Methyl-p-[2-(phenethoxy)ethoxy]-phenethyl]imino]bis[(S)-3-phenoxy-2-propanol] and (S)-1-[[(R)-α-methyl-p-[2-(ethoxy)ethoxy]phenethyl]-amino]-3-phenoxypropanol.

14. Methyl (E)-p-[(R)-2-[bis[(RS)-2-hydroxy-3-phenoxypropyl]amino]propy]-β-methyl-cinnamate,
methyl p-[(R)-2-[bis[(RS)-2-hydroxy-3-phenoxypropyl]-amino]propyl]benzoate,
6-[p-(R)-[bis[(S)-2-hydroxy-3-phenoxypropyl]amino]-propyl]phenoxy-1-hexanol,
methyl (E)-p-[(R)-2-[[(RS)-2-hydroxy-3-phenoxypropyl]-amino]propyl]-β-methyl-cinnamate,
p-[(R)-3-[[(RS)-2-hydroxy-3-phenoxypropyl]amino]butyl]-benzamide,
methyl p-[(R)-2-[[(RS)-2-hydroxy-3-phenoxypropyl]-amino]propyl]benzoate.
p-[(R)-2-[[(S)-2-hydroxy-3-phenoxypropyl]amino]propyl]-phenol and
6-[p-[(R)-2-[[(S)-2-hydroxy-3-phenoxypropyl]amino]-propyl]phenoxy]hexanol.

15. Compounds according to claims 1-14, as well as compounds of formula V-1 in which Z is phenyl substituted by lower-alkoxy or by a group $-O-(CH_2)_q-COOR^4$ or $-O-(CH_2)_q-O-(CH_2)_t-R^5$, wherein $R^4$ is lower-alkyl and $R^5$ is phenyl para-substituted by chlorine, fluorine, trifluoromethyl, lower-alkyl or lower-alkoxy, as therapeutically active agents, especially as agents for the treatment of obesity and of diabetes mellitus or of conditions which are associated with an increased protein breakdown.

16. Compounds according to claims 1-14, as well as compounds of formula V-1 in which Z is phenyl substituted by lower-alkoxy or by a group $-O-(CH_2)_q-COOR^4$ or $-O-(CH_2)_q-O-(CH_2)_t-R^5$, wherein $R^4$ is lower-alkyl and $R^5$ is phenyl para-substituted by chlorine, fluorine, trifluoromethyl, lower alkyl or lower-alkoxy, as feed additives.

17. A medicament, especially for the treatment of obesity and of diabetes mellitus or of conditions which are associated with an increased protein breakdown, containing a compound according to any one of claims 1-14 or a compound of formula V-1 in which Z is phenyl substituted by lower-alkoxy or by a group $-O-(CH_2)_q-COOR^4$ or $-O-(CH_2)_q-O-(CH_2)_t-R^5$, wherein $R^4$ is lower-alkyl and $R^5$ is phenyl parasubstituted by chlorine, fluorine, trifluoromethyl, lower-alkyl or lower-alkoxy.

18. The use of compounds according to claims 1-14 as well as compounds of formula V-1 in which Z is phenyl substituted by lower-alkoxy or by a group $-O-(CH_2)_q-COOR^4$ or $-O-(CH_2)_q-O-(CH_2)_t-R^5$, wherein $R^4$ is lower-alkyl and $R^5$ is phenyl parasubstituted by chlorine, fluorine, trifluoromethyl, lower-alkyl or lower-alkoxy, in the manufacture of medicaments for the treatment of obesity and of diabetes mellitus or of conditions which are associated with an increased protein breakdown.

**Claims** (for the Contracting State AT)

1. A process for the manufacture of phenoxypropanolamines of the formula

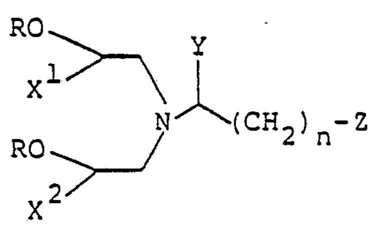

(I)

wherein

n is the number 1 or 2,

R is hydrogen, lower-alkanoyl or phenyl-lower-alkanoyl,

$X^1$ is phenoxymethyl optionally mono-fluorinated or mono-chlorinated in the ortho-position,

$X^2$ is lower-alkyl, phenoxymethyl optionally monofluorinated or mono-chlorinated in the orthoposition or phenyl optionally monosubstituted by fluorine, chlorine, trifluoromethyl or lower-alkoxy,

Y is hydrogen or methyl,

Z is a group of the formula

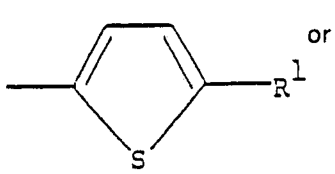

or

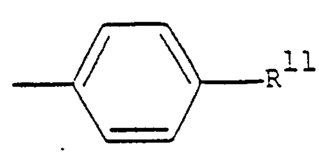

$R^1$ is optionally N-mono-lower-alkylated or N-di-lower-alkylated aminomethyl or a residue $-C(O)R^2$, $-C(R^3)=CH-(CH_2)_m-C(O)R^2$, $-C(H,R^3)-(CH_2)_{m+1}-C(O)R^2$, $-C(H,R^3)-(CH_2)_p-OH$ or $-C(R^3)=CH-C(CH_3)=CH-COOCH_3$,

$R^{11}$ is hydroxy, lower-alkanoyloxy, sulphamoyl, benzyloxy or phenoxy optionally ring-substituted by fluorine, chlorine, trifluoromethyl, lower-alkyl or lower-alkoxy, or a group $R^1$, $-C-(CH_2)_q-OH$, $-O-(CH_2)_q-O-(CH_2)_t-R^5$ or

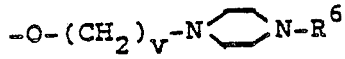

$R^2$ is hydroxy, lower-alkyl, lower-alkoxy, dimethylaminoethoxy, lower-alkoxycarbonylethyl or optionally mono-lower alkylated or di-lower alkylated amino,

$R^3$ is hydrogen or methyl,

$R^5$ is hydrogen, lower-alkyl or phenyl,

$R^6$ is lower-alkyl or phenyl optionally para-substituted by fluorine, chlorine, lower-alkyl or lower-alkoxy,

m and p are whole numbers of 0 to 6,

v is a whole number of 2 to 4,

q and t are whole numbers of 1 to 6,

and of physiologically compatible salts thereof, characterized by

a) reacting an epoxide of the formula

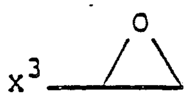

(II)

or a β-keto halide of the formula

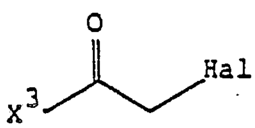

(III)

with a primary amine of the formula

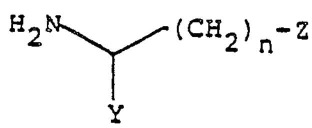

(IV)

or a secondary amine of the formula

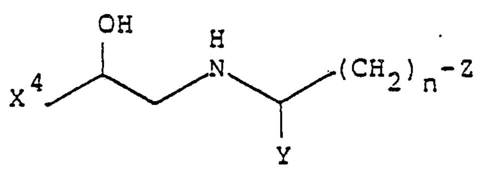

(V)

wherein in formulae II-V n, Y and Z the above significance, Hal is halogen, one of the groups $X^3$ and $X^4$ is a group $X^1$ and the other is a group $X^2$, whereby further $X^3$ is a group $X^1$ when a compound of formula II or III is reacted with a compound of formula IV,

and reducing a $X^1-C(O)-$ or $X^2-C(O)-$ group in a compound obtained to a $X^1-CHOH-$ or $X^2-CHOH-$ group,

b) if desired, functionally modifying a reactive substituent present in a group Z of a compound of formula I,

c) if desired, alkanoylating or phenalkanoylating the hydroxy groups present in a diol of formula I, and

d) if desired, converting a compound of formula I into a salt.

2. A process for the manufacture of phenoxypropanolamines of the formula

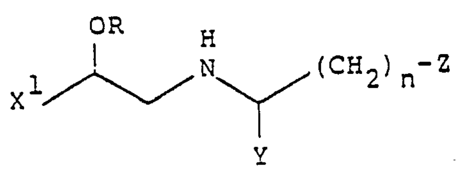

V-1

wherein $X^1$, R, Y, Z and n have the significance given earlier, and of physiologically compatible salts thereof, characterized by

a) reacting an epoxide of the formula

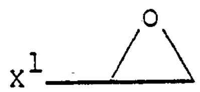

II-1

or a β-keto halide of the formula

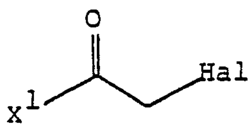

III-1

with an amine of formula IV and reducing a $X^1$—C(O)— group present in a compound obtained to the $X^1$—CH(OH)— group, or

b) reducing a compound of one of the formulae

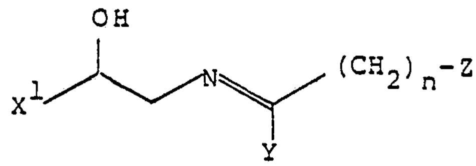

(VI)

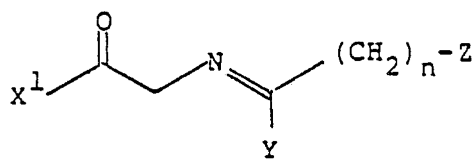

(VII)

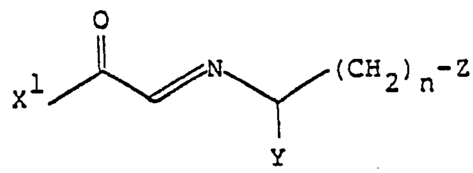

(VIII)

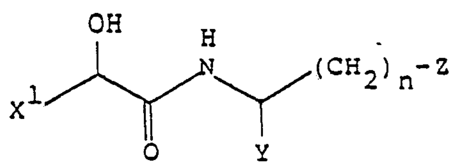

(IX)

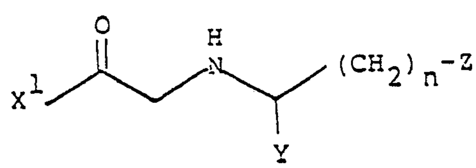

(X)

c) if desired, functionally modifying a reactive substituent present in a group Z of a compound of formula V-1,

d) if desired, alkanoylating or phenalkanoylating the hydroxy group present in an alcohol of formula V-1, and

e) if desired, converting a compound of formula V-1 into a salt,

wherein in the previous formulae the residues $X^1$, Y, Z and n have the significance given earlier.

3. A process for the manufacture of compounds according to claim 1 or 2, wherein the substituent $R^{11}$ present on a phenyl group $R^{11}$ is hydroxy, lower-alkanoyloxy, sulphamoyl or a group $R^1$, $-O-(CH_2)_q-OH$ or $-O-(CH_2)_q-O-(CH_2)_t-R^5$ and the remaining symbols have the same significance as in claim 1 or 2, characterized in that correspondingly substituted starting materials are used.

4. A process for the manufacture of compounds according to any one of claims 1-3, wherein R is hydrogen, characterized in that correspondingly substituted starting materials are used.

5. A process for the manufacture of compounds according to any one of claims 1-4, wherein $X^1$ is phenoxymethyl and $X^2$ is phenoxymethyl or phenyl, especially those in which the C-atom bonded to a phenoxymethyl residue $X^1$ or $X^2$ has the S-configuration or the C-atom bonded to a phenyl residue $X^2$ has the R-configuration, characterized in that correspondingly substituted starting materials are used.

6. A process for the manufacture of compounds according to any one of claims 1-5, wherein Y is methyl, especially those in which the C-atom bonded to a methyl residue Y has the R-configuration, characterized in that correspondingly substituted starting materials are used.

7. A process for the manufacture of compounds according to any one of claims 1-6, wherein Z is phenyl or thienyl substituted by carbamoyl, methoxycarbonyl or 2-(ethoxy or methoxy)-carbonyl-1-methylvinyl, characterized in that correspondingly substituted starting materials are used.

8. A process for the manufacture of compounds according to any one of claims 1-6, wherein Z is phenyl substituted by 6-hydroxyhexoxy or 2-phenethoxy-2-ethoxy, characterized in that correspondingly substituted starting materials are used.

9. A process for the manufacture of compounds according to any one of claims 2-6, wherein Z is p-hydroxyphenyl, characterized in that correspondingly substituted starting materials are used.

10. A process for the manufacture of compounds according to any one of claims 1-8, wherein R is hydrogen, $X^1$ is phenoxymethyl, $X^2$ is phenoxymethyl or phenyl, Y is methyl and Z is phenyl or thienyl substituted by carbamoyl, methoxycarbonyl or 2-(ethoxy or methoxy)-carbonyl-1-methylvinyl, or phenyl substituted by 6-hydroxyhexoxy or 2-phenethoxy-2-ethoxy, characterized in that correspondingly substituted starting materials are used.

11. A process for the manufacture of compounds according to any one of claims 2-6 and 9, wherein R is hydrogen, $X^1$ is phenoxymethyl, Y is methyl and Z is p-hydroxyphenyl, characterized in that correspondingly substituted starting materials are used.

12. A process for the manufacture of compounds according to any one of claims 1-11, wherein the C-atom bonded to a methyl residue Y has the R-configuration, the C-atom bonded to a phenoxymethyl residue $X^1$ or $X^2$ has the S-configuration and the C-atom bonded to a phenyl residue $X^2$ has the R-configuration, characterized in that correspondingly substituted starting materials are used.

13. A process according to claim 1 or 2 for the manufacture of 1,1'-[[(R)-α-methyl-p-[2-(phenethoxy)-ethoxy] phenethyl] imino] bis[(S)-3-phenoxy-2-propanol] or of (S)-1-[[(R)-α-methyl-p-[2-(ethoxy) ethoxy]phenethyl] amino]-3-phenoxypropanol, characterized in that correspondingly substituted starting materials are used.

14. A process according to claim 1 or 2 for the manufacture of methyl (E)-p-[(R)-2-[bis[(RS)-2-hydroxy-3-phenoxypropyl] amino] propyl]-β-methyl-cinnamate,
methyl p-[(R)-2-[bis[(RS)-2-hydroxy-3-phenoxypropyl]-amino] propyl] benzoate,
6-[p-[(R)-bis[(S)-2-hydroxy-3-phenoxypropyl] amino]-propyl] phenoxy-1-hexanol,
methyl (E)-p-[(R)-2-[[(RS)-2-hydroxy-3-phenoxypropyl]-amino] propyl]-β-methyl-cinnamate,
p-[(R)-3-[[(RS)-2-hydroxy-3-phenoxypropyl] amino] butyl]-benzamide,
methyl p-[(R)-2-[[(RS)-2-hydroxy-3-phenoxypropyl]-amino] propyl] benzoate,
p-[(R)-2-[[(S)-2-hydroxy-3-phenoxypropyl] amino] propyl]-phenol or
of 6-[p-[(R)-2-[[(S)-2-hydroxy-3-phenoxypropyl] amino]-propyl] phenoxy] hexanol,
characterized in that correspondingly substituted starting materials are used.

**Revendications** (pour les Etats contractants : BE, CH, DE, FR, GB, IT, LI, LU, NL, SE)

1. Phénoxypropanolamines de formule

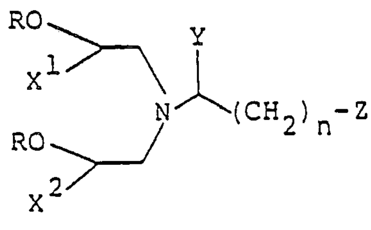

(I)

où

n représente le nombre 1 ou 2 ;

R représente un hydrogène, alcanoyle inférieur ou phényl-alcanoyle inférieur,

$X^1$ représente un phénoxyméthyle éventuellement monofluoré ou monochloré en position ortho,

$X^2$ représente un alcoyle inférieur, un phénoxyméthyle éventuellement monofluoré ou monochloré en position ortho ou un phényle éventuellement monosubstitué par un fluor, un chlore, un trifluorométhyle ou un alcoxy inférieur,

Y représente un hydrogène ou un méthyle,

Z représente un groupe de formule

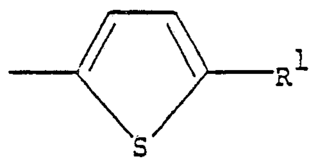

ou

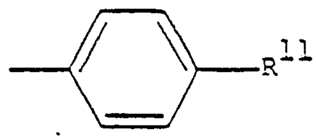

$R^1$ représente un aminométhyle éventuellement N-mono ou N-disubstitué par un alcoyle inférieur ou un radical $-C(O)R^2$,

$-C(R^3) = CH-(CH_2)_m-C(O)R^2$,

$-C(H,R^3)-(CH_2)_{m+1}-C(O)R^2$,

$-C(H,R^3)-(CH_2)_p-OH$ ou

$-C(R^3) = CH-C(CH_3) = CH-COOCH_3$,

$R^{11}$ représente un hydroxy, alcanoyloxy inférieur, sulfamoyle, benzyloxy ou phénoxy éventuellement substitué dans son noyau par un fluor, chlore, trifluorométhyle, alcoyle inférieur ou alcoxy inférieur ou un groupe $R^1$, $-O-(CH_2)_q-OH$,

$-O-(CH_2)_q-O-(CH_2)_t-R^5$ ou

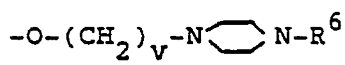

$R^2$ représente un hydroxy, alcoyle inférieur, alcoxy inférieur, diméthylaminoéthoxy, alcoxy inférieur-carbonyléthyle ou amino éventuellement mono- ou disubstitué par un alcoyle inférieur,

$R^3$ représente un hydrogène ou un méthyle,

$R^5$ représente un hydrogène, alcoyle inférieur ou phényle,

$R^6$ représente un alcoyle inférieur ou un phényle éventuellement substitué en position para par un fluor, un chlore, un alcoyle inférieur ou un alcoxy inférieur,

m et p représentent des nombres entiers allant de 0 à 6,

v représente un nombre entier allant de 2 à 4,

q et t représentent des nombres entiers allant de 1 à 6,

et leurs sels physiologiquement acceptables.

2. Phénoxypropanolamines de formule

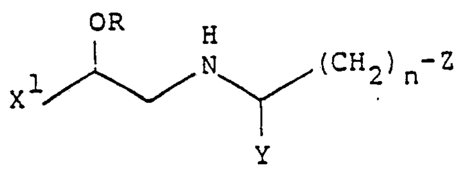

V-1

où $X^1$, R, Y, Z et n ont la signification donnée ci-dessus,

et leurs sels physiologiquement acceptables.

3. Composés selon la revendication 1 ou 2, où le substituant $R^{11}$ contenu dans un groupe phényle Z est un hydroxy, alcanoyloxy inférieur, sulfamoyle, ou un groupe $R^1$, $-O-(CH_2)_q-OH$ ou $-O-(CH_2)_q-O-(CH_2)_t-R^5$ et les autres symboles ont la même signification que dans la revendication 1 ou 2.

4. Composés selon l'une des revendications 1-3, où R est un hydrogène.

5. Composés selon l'une des revendications 1-4 où $X^1$ est un phénoxyméthyle et $X^2$ un phénoxyméthyle ou un phényle, en particulier ceux où l'atome de carbone lié à un radical phénoxyméthyle $X^1$ ou $X^2$ a la configuration S, ou bien où l'atome de carbone lié à un radical phényle $X^2$ a la configuration R.

6. Composés selon l'une des revendications 1-5, où Y est un méthyle, en particulier ceux où l'atome de carbone lié à un radical méthyle Y a la configuration R.

7. Composés selon l'une des revendications 1-6, où Z est un phényle ou un thiényle substitué par un carbamoyle, méthoxycarbonyle ou 2-(éthoxy ou méthoxy)-carbonyl-1-méthylvinyle.

8. Composés selon l'une des revendications 1-6, où Z est un phényle substitué par un 6-hydroxyhexoxy ou 2-phénéthoxy-2-éthoxy.

9. Composés selon l'une des revendications 2-6, où Z est un p-hydroxyphényle.

10. Composés selon l'une des revendications 1-8, où R représente un hydrogène, $X^1$ un phénoxymé-

thyle, $X^2$ un phénoxyméthyle ou un phényle, Y un méthyle et Z un phényle ou un thiényle substitué par un carbamoyle, méthoxycarbonyle ou 2-(éthoxy ou méthoxy)-carbonyl-1-méthylvinyle, ou un phényle substitué par un 6-hydroxyhexoxy ou 2-phénéthoxy-2-éthoxy.

11. Composés selon l'une des revendications 2-6 et 9, où R est un hydrogène, $X^1$ est un phénoxyméthyle, Y un méthyle et Z un p-hydroxyphényle.

12. Composés selon l'une des revendications 1-11, où l'atome de carbone lié au radical méthyle Y à la configuration R, l'atome de carbone lié à un radical phénoxyméthyle $X^1$ ou $X^2$ a la configuration S et l'atome de carbone lié à un radical phényle $X^2$ a la configuration R.

13. 1,1'-[[(R)-α-méthyl-p-[2-(phénéthoxy) éthoxyl-phénéthyl] imino] bis[(S)-3-phénoxy-2-propanol] et (S)-1-[[(R)-α-méthyl-p-[2-(éthoxy) éthoxy] phénéthyl] amino]-3-phénoxypropanol.

14. Ester méthylique de l'acide (E)-p[(R)-2-[Bis[(RS)-2-hydroxy-3-phénoxy-propyl] amino] propyl]-β-méthyl-cinnamique,

Ester méthylique de l'acide p-[(R)-2-[Bis[(RS)-2-hydroxy-3-phénoxypropyl] amino]-propyl] benzoïque,

6-[p-(R)-[Bis[(S)-2-hydroxy-3-phénoxypropyl] amino]-propyl] phénoxy-1-hexanol,

Ester méthylique de l'acide (E)-[(R)-2-[[(RS)-2-hydroxy-3-phénoxypropyl]-amino] propyl]-β-méthyl-cinnamique,

p-[(R)-3-[[(RS)-2-hydroxy-3-phénoxypropyl] amino]-butyl] benzamide,

Ester méthylique de l'acide p-[(R)-2-[[(RS)-2-hydroxy-3-phénoxypropyl] amino]-propyl] benzoïque, p-[(R)-2-[[(S)-2-hydroxy-3-phénoxypropyl] amino]-propyl] phénol et

6-[p-[(R)-2-[[(S)-2-hydroxy-3-phénoxypropyl] amino]-propyl] phénoxy] hexanol.

15. Composés selon les revendications 1-14, ainsi que composés de formule V-1, où Z est un phényle substitué par un alcoxy inférieur ou par un groupe $-O-(CH_2)_q-COOR^4$ ou $-O-(CH_2)_t-O-CH_2)_t-R^5$, où $R^4$ est un alcoyle inférieur et $R^5$ est un phényle substitué en position para par un chlore, fluor, trifluorométhyle, alcoyle inférieur ou alcoxy inférieur, comme agent à action thérapeutique, en particulier comme agent pour le traitement de l'adipose et du diabète sucré ou des états qui sont liés à un accroissement de la dégradation des protéines.

16. Composés selon les revendications 1-14, ainsi que composés de formule V-1, où Z est un phényle substitué par un alcoxy inférieur ou par un groupe $-O(CH_2)_q-COOR^4$ ou $-O-(CH_2)_q-O-(CH_2)_t-R^5$, où $R^4$ est un alcoyle inférieur et $R^5$ un phényle substitué en position para par un chlore, fluor, trifluorométhyle, alcoyle inférieur ou alcoxy inférieur, comme additif alimentaire.

17. Médicament, en particulier pour le traitement de l'adipose et du diabète sucré ou des états qui sont liés à un accroissement de la dégradation des protéines, contenant un composé selon l'une des revendications 1-14 ou un composé de formule V-1, où Z est un phényle substitué par un alcoxy inférieur ou par un groupe $-O-(CH_2)_q-COOR^4$ ou $-O-(CH_2)_q-O-(CH_2)_t-R^5$, où $R^4$ est un alcoyle inférieur et $R^5$ est un phényle substitué en para par un chlore, fluor, trifluorométhyle, alcoyle inférieur ou alcoxy inférieur.

18. Application de composés selon les revendications 1-14 ainsi que de composés de formule V-1, où Z est un phényle substitué par un alcoxy inférieur ou par un groupe $-O-(CH_2)_q-COOR^4$ ou $-O-(CH_2)_q-O-(CH_2)_t-R^5$, où $R^4$ est un alcoyle inférieur et $R^5$ un phényle substitué en position para par un chlore, fluor, trifluorométhyle, alcoyle inférieur ou alcoxy inférieur, à la préparation de médicaments pour le traitement de l'adipose et du diabète sucré ou des états qui sont liés à un accroissement de la dégradation des protéines.

**Revendications** (pour l'Etat contractant AT)

1. Procédé de préparation de phénoxypropanolamines de formule

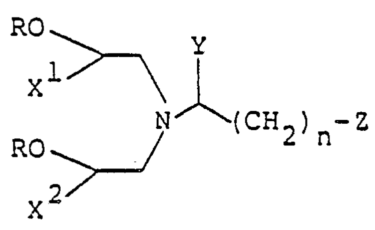

(I)

où

n représente le nombre 1 ou 2 ;

R représente un hydrogène, alcanoyle inférieur ou phényl-alcanoyle inférieur,

$X^1$ représente un phénoxyméthyle éventuellement monofluoré ou monochloré en position ortho,

$X^2$ représente un alcoyle inférieur, un phénoxyméthyle éventuellement monofluoré ou monochloré en position ortho ou un phényle éventuellement monosubstitué par un fluor, un chlore, un trifluorométhyle ou un alcoxy inférieur,

Y représente un hydrogène ou un méthyle,

Z représente un groupe de formule

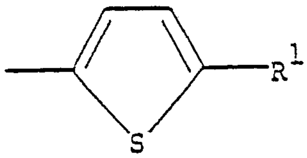

ou

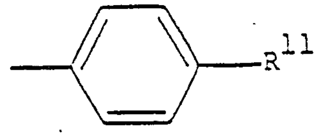

$R^1$ représente un aminométhyle éventuellement N-mono ou N-disubstitué par un alcoyle inférieur ou un radical $—C(O)R^2$,
$—C(R^3) = CH—(CH_2)_m—C(O)R^2$,
$—C(H,R^3)—(CH_2)_{m+1}—C(O)R^2$,
$—C(H,R^3)—(CH_2)_p—OH$ ou
$—C(R^3) = CH—C(CH_3) = CH—COOCH_3$

$R^{11}$ représente un hydroxy, alcanoyloxy inférieur, sulfamoyle, benzyloxy ou phénoxy éventuellement substitué dans son noyau par un fluor, chlore, trifluorométhyle, alcoyle inférieur ou alcoxy inférieur ou un groupe $R^1$, $—O—(CH_2)_q—OH$,
$—O—(CH_2)_q—O—(CH_2)_t—R^5$ ou

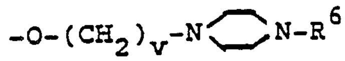

$R^2$ représente un hydroxy, alcoyle inférieur, alcoxy inférieur, diméthylaminoéthoxy, alcoxy inférieur carbonyléthyle ou amino éventuellement mono- ou disubstitué par un alcoyle inférieur,
$R^3$ représente un hydrogène ou un méthyle,
$R^5$ représente un hydrogène, alcoyle inférieur ou phényle,
$R^6$ représente un alcoyle inférieur ou un phényle éventuellement substitué en position para par un fluor, un chlore, un alcoyle inférieur ou un alcoxy inférieur,
m et p représentent des nombres entiers allant de 0 à 6,
v représente un nombre entier allant de 2 à 4,
q et t représentent des nombres entiers allant de 1 à 6,
et leurs sels physiologiquement acceptables, caractérisé en ce que

a) on fait réagir un époxyde de formule

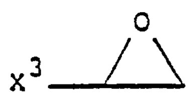

(II)

ou un β-cétohalogénure de formule

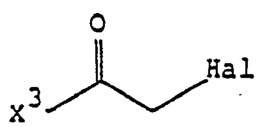

(III)

avec une amine primaire de formule

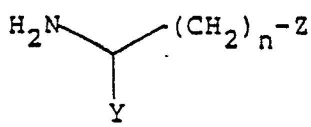

(IV)

ou une amine secondaire de formule

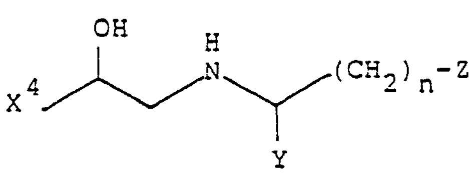

(V)

où dans les formules II-V n, Y et Z ont la signification donnée ci-dessus. Hal représente un halogène, l'un des groupes $X^3$ et $X^4$ un groupe $X^1$ et l'autre un groupe $X^2$, où $X^3$ est un groupe $X^1$ si l'on fait réagir un composé de formule II ou III avec un composé de formule IV,
et on réduit un groupe $X^1—C(O)—$ ou $X^2—C(O)—$ présent dans un composé obtenu en un groupe $X^1—CHOH$ ou $X^2—CHOH—$,

b) si on le désire, on fait subir une modification fonctionnelle à un substituant réactif contenu dans un groupe Z d'un composé de formule I,

c) si on le désire on alcanoyle ou phénylalcanoyle les groupes hydroxy présents dans un diol de formule I et

d) si on le désire, on transforme un composé de formule I en un sel.

2. Procédé de préparation de phénoxypropanolamines de formule

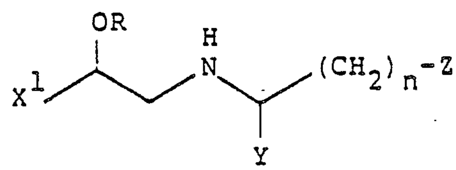

V-1

où $X^1$, R, Y, Z et n ont la signification donnée ci-dessus, et de leurs sels physiologiquement acceptables, caractérisé en ce que

a) on fait réagir un époxyde de formule

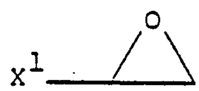

II-1

ou un β-cétohalogénure de formule

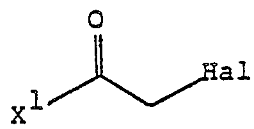

III-1

avec une amine de formule IV et on réduit un groupe $X^1$—C(O)— présent dans un composé obtenu en un groupe $X^1$—CH(OH)—, ou

b) on réduit un composé d'une des formules

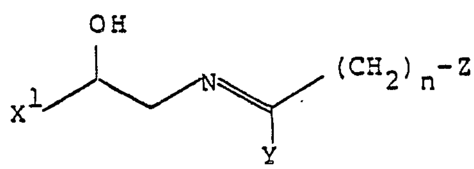

(VI)

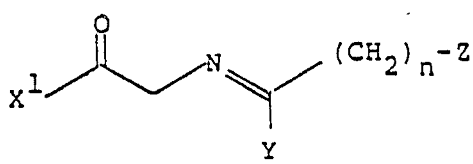

(VII)

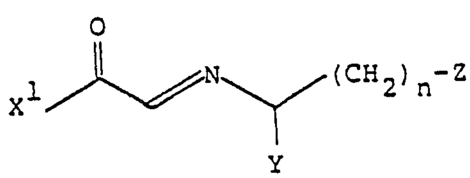

(VIII)

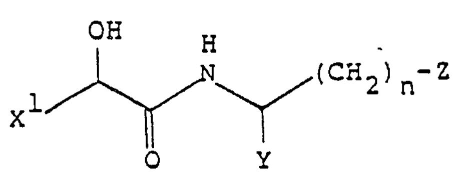

(IX)

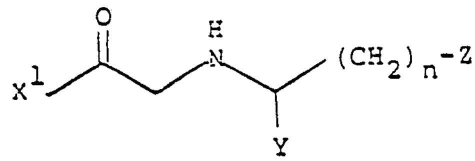

(X)

c) si on le désire, on fait subir une modification fonctionnelle à un substituant réactif contenu dans un groupe Z d'un composé de formule V-1,

d) si on le désire, on alcanoyle ou phénalcanoyle le groupe hydroxy présent dans un alcool de formule V-1, et

e) si on le désire, on transforme un composé de formule V-1 en un sel,

où dans les formules ci-dessus, les radicaux $X^1$, Y, Z et n ont la signification donnée ci-dessus.

3. Procédé de préparation de composés selon la revendication 1 ou 2, où le substituant $R^{11}$ contenu dans un groupe phényle Z est un hydroxy, alcanoyloxy inférieur, sulfamoyle ou un groupe $-O-(CH_2)_q-OH$ ou $-O-(CH_2)_q-O-(CH_2)_t-R^5$ et les autres symboles ont la même signification que dans la revendication 1 ou 2, caractérisé en ce qu'on utilise des produits de départ substitués de façon correspondante.

4. Procédé de préparation de composés selon l'une des revendications 1-3, où R est un hydrogène, caractérisé en ce qu'on utilise des produits de départ substitués de façon correspondante.

5. Procédé de préparation de composés selon l'une des revendications 1-4, où $X^1$ est un phénoxyméthyle et $X^2$ un phénoxyméthyle ou un phényle, en particulier ceux où l'atome de carbone lié à un radical phénoxyméthyle $X^1$ ou $X^2$ a la configuration S ou bien l'atome de carbone lié à un radical phényle $X^2$ a la configuration R, caractérisé en ce qu'on utilise des produits de départ substitués de façon correspondante.

6. Procédé de préparation de composés selon l'une des revendications 1-5, où Y est un méthyle, en particulier ceux où l'atome de carbone lié sur un radical méthyle Y a la configuration R, caractérisé en ce qu'on utilise des produits de départ substitués de façon correspondante.

7. Procédé de préparation de composés selon l'une des revendications 1-6, où Z est un phényle ou un thiényle substitué par un carbamoyle, méthoxycarbonyle ou 2-(éthoxy ou méthoxy)-carbonyl-1-méthylvinyle caractérisé en ce qu'on utilise des produits de départ substitués de façon correspondante.

8. Procédé de préparation de composés selon l'une des revendications 1-6, où Z est un phényle substitué par 6-hydroxyhexoxy ou 2-phénéthoxy-2-éthoxy, caractérisé en ce qu'on utilise des produits de départ substitués de façon correspondante.

9. Procédé de préparation de composés selon l'une des revendications 2-6, où Z est un p-hydroxyphényl, caractérisé en ce qu'on utilise des produits de départ substitués de façon correspondante.

10. Procédé de préparation de composés selon l'une des revendications 1-8, où R représente un hydrogène, $X^1$ un phénoxyméthyle, $X^2$ un phénoxyméthyle ou un phényle, Y un méthyle et Z un phényle ou thiényle substitué par un carbamoyle, méthoxycarbonyle ou 2-(éthoxy ou méthoxy)-carbonyl-1-méthylvinyle, ou un phényle substitué par 6-hydroxyhexoxy ou 2-phénéthoxy-2-éthoxy, caractérisé en ce qu'on utilise des produits de départ substitués de façon correspondante.

11. Procédé de préparation de composés selon l'une des revendications 2-6 et 9, où R est un hydrogène, $X^1$ un phénoxyméthyle, Y un méthyle et Z un p-hydroxyphényle, caractérisé en ce qu'on utilise des produits de départ substitués de façon correspondante.

12. Procédé de préparation de composés selon l'une des revendications 1-11, où l'atome de carbone lié sur un radical méthyle Y a la configuration R, l'atome de carbone lié à un radical phénoxyméthyle $X^1$ ou $X^2$ a la configuration S et l'atome de carbone lié à un radical phényle $X^2$ a la configuration R, caractérisé en ce qu'on utilise des produits de départ substitués de façon correspondante.

13. Procédé selon la revendication 1 ou 2 pour la préparation du 1,1'-[[(R)-α-méthyl-p-[2-(phénéthoxy) éthoxy] phénéthyl] imino] bis[(S)-3-phénoxy-2-propanol] ou du (S)-1-[[(R)-α-méthyl-p-[2-(éthoxy) éthoxy] phénéthyl] amino]-3-phénoxypropanol, caractérisé en ce qu'on utilise des produits de départ substitués de façon correspondante.

14. Procédé selon la revendication 1 ou 2 pour la préparation de l'ester méthylique de l'acide (E)-p-[(R)-2-[Bis-[(RS)-2-hydroxy-3-phénoxypropyl] amino] propyl]-β-méthyl-cinnamique,

l'ester méthylique de l'acide p-[(R)-2-[Bis[(RS)-2-hydroxy-3-phénoxypropyl] amino]-propyl] benzoïque,

le 6-[p-(R)-[Bis[(S)-2-hydroxy-3-phénoxypropyl] amino]-propyl] phénoxy-1-hexanol,

l'ester méthylique de l'acide (E)-p-[(R)-2-[[(RS)-2-hydroxy-3-phénoxypropyl]-amino] propyl]-β-méthylcinnamique,

le p-[(R)-3-[[(RS)-2-hydroxy-3-phénoxypropy] amino]-butyl] benzamide,

l'ester méthylique de l'acide p-[(R)-2-[[(RS)-2-hydroxy-3-phénoxypropyl] amino]-propyl] benzoïque,

le p-[(R)-2-[[(S)-2-hydroxy-3-phénoxypropyl] amino]-propyl] phénol ou le

6-[p-[(R)-2-[[(S)-2-hydroxy-3-phénoxypropyl]-amino] propyl] phénoxy] hexanol,

caractérisé en ce qu'on utilise des produits de départ substitués de façon correspondante.